# EUROPEAN PATENT APPLICATION

(11) **EP 4 667 480 A1**
(43) Date of publication of application: **24.12.2025**
(21) Application number: 24756899.1
(22) Date of filing: 14.02.2024
(51) Int. Cl.: C07K 1/00, C12N 15/11, C40B 40/06, C40B 40/10

(54) **TRANSLATION INITIATION WITH EXOTIC AMINO ACIDS USING EF-P-RESPONSIVE ARTIFICIAL INITIATOR TRNA**

(30) Priority: 16.02.2023 JP 2023022106
(71) Applicant: The University of Tokyo, Bunkyo-ku, Tokyo 113-8654 (JP)
(72) Inventor: SUGA, Hiroaki, Tokyo 113-8654 (JP); KATOH, Takayuki, Tokyo 113-8654 (JP)
(74) Representative: D Young & Co LLP
(86) International application number: PCT/JP2024/004960
(87) International publication number: WO 2024/172058

(57) **Abstract**

A production method for a peptide comprising translating in a cell-free translation system using an initiator tRNA comprising a nucleotide sequence set forth in SEQ ID NO: 1,
GCGCN₁N₂N₃N₄N₅N₆N₇N₈N₉GCGC (SEQ ID NO: 1)
(in SEQ ID NO: 1, N₁ to N₉ each independently represent any base, N₁ to N₉ form a D-loop, and GCGC forms a base pair with GCGC).

## Description

### Technical Field

The present invention relates to translation initiation with exotic amino acids using an EF-P-responsive artificial initiator tRNA. Specifically, the present invention relates to a production method for a peptide, an initiator tRNA, a translation system, a method for producing a peptide library, a method for producing a peptide-mRNA complex library, and the like.

### Background Art

In translation initiation, the N-formylmethionyl initiator tRNA (fMet-tRNAⁱⁿⁱ) at the P site is recognized by the translation initiation factor IF3, and positioned at an active site through the evaluation of the stability of the codon-anticodon interaction (Non-Patent Literatures 1 to 3). In a case where the fMet-tRNAⁱⁿⁱ is not positioned at the active site by IF3, the fMet-tRNAⁱⁿⁱ is finally fallen off from the ribosome, translation is reinitiated from the second aminoacyl tRNA at the A site, and a peptide lacking the N-terminal fMet (N-formylmethionine), referred to as a reinitiated peptide (RiP), is synthesized (Non-Patent Literature 4). This phenomenon is referred to as N-terminal drop-off-reinitiation.

By the reprogramming of the gene code, not only fMet but also various non-standard substrates can be artificially introduced at the N terminus of the peptide. However, the incorporation of a non-standard substrate having a low peptidyl donor activity, such as N-acetyl-L-proline (AcPro), more frequently causes N-terminal drop-off-reinitiation (Non-Patent Literatures 4 and 5). Therefore, it is very difficult to introduce such a non-proteinogenic amino acid at the N terminus.

In addition, in nature, the ribosomal translation system uses 19 proteinogenic L-amino acids and glycine, but non-proteinogenic amino acids such as D-amino acids, β-amino acids, and γ-amino acids are excluded from peptide synthesis and protein synthesis by the ribosomal translation system. D-amino acids and β-amino acids are found in various natural peptides, but are usually synthesized by non-ribosomal synthetic pathways. Therefore, various methods for introducing these non-proteinogenic amino acids have been developed.

For example, Patent Literature 1 discloses a translation system capable of synthesizing a peptide in which non-proteinogenic amino acids are consecutively incorporated, in a case of translating in a cell-free translation system. Patent Literature 2 discloses a method for synthesizing a peptide that enables simultaneous incorporation of a plurality of different N-methyl amino acids.

### Citation List

### Patent Literatures

Patent Literature 1: Pamphlet of International Publication No. 2019/077887
Patent Literature 2: Pamphlet of International Publication No. 2021/100833
Non-Patent Literature 1: Simonetti, A., Marzi, S., Myasnikov, A.G., Fabbretti, A., Yusupov, M., Gualerzi, C.O. and Klaholz, B.P. (2008) Structure of the 30S translation initiation complex. Nature, 455, 416-420.
Non-Patent Literature 2: Hussain, T., Llacer, J.L., Wimberly, B.T., Kieft, J.S. and Ramakrishnan, V. (2016) Large-scale movements of IF3 and tRNA during bacterial translation initiation. Cell, 167, 133-144.e113.
Non-Patent Literature 3: Kaledhonkar, S., Fu, Z., Caban, K., Li, W., Chen, B., Sun, M., Gonzalez, R.L. and Frank, J. (2019) Late steps in bacterial translation initiation visualized using time-resolved cryo-EM. Nature, 570, 400-404.
Non-Patent Literature 4: Katoh, T. and Suga, H. (2022) Drop-off-reinitiation at the N-termini of nascent peptides and its regulation by IF3, EF-G, and RRF. Nucleic Acids Res..
Non-Patent Literature 5: Muto, H. and Ito, K. (2008) Peptidyl-prolyl-tRNA at the ribosomal P-site reacts poorly with puromycin. Biochem. Biophys. Res. Commun., 366, 1043-1047.

### Summary of Invention

### Technical Problem

However, since the initiation reaction and the elongation reaction are clearly different in the mechanism of translation, the elongator tRNA as described in Patent Literatures 1 and 2 cannot be used for the initiation of translation.

Therefore, there is a demand for a novel method for introducing various amino acids comprising non-proteinogenic amino acids such as acetyl-L-proline (AcPro), D-amino acids, β-amino acids, and γ-amino acids, at the N terminus of a peptide.

Accordingly, an object to be solved by the present invention is to provide a production method for a peptide, which enables various amino acids to be introduced efficiently and in a versatile manner at an N terminus of a peptide, and an initiator tRNA and a translation system for use in such a production method.

### Solution to Problem

As a result of intensive studies, the inventors of the present invention have found that by translating in a cell-free translation system using an initiator tRNA having a predetermined structure, N-terminal drop-off-reinitiation can be suppressed and translation initiation using various amino acids at the N terminus can be achieved, thereby completing the present invention.

That is, the present invention is as follows.
[1] A production method for a peptide comprising:
   translating in a cell-free translation system using an initiator tRNA containing a nucleotide sequence set forth in SEQ ID NO: 1,
   GCGCN₁N₂N₃N₄N₅N₆N₇N₈N₉GCGC (SEQ ID NO: 1)
   (in SEQ ID NO: 1, N₁ to N₉ each independently represent any base, N₁ to N₉ form a D-loop, and GCGC forms a base pair with GCGC).
[2] The production method according to [1], wherein the peptide to be produced is a peptide comprising, at an N terminus, a D-amino acid, a β-amino acid, a γ-amino acid, or L-proline, each comprising a nitrogen atom modified at an α-position, a β-position, or a γ-position.
[3] The production method according to [1] or [2], wherein the initiator tRNA is charged with a D-amino acid, a β-amino acid, a γ-amino acid, or L-proline, each comprising a nitrogen atom modified at an α-position, a β-position, or a γ-position, or with a peptide.
[4] The production method according to any one of [1] to [3], wherein the initiator tRNA further contains a nucleotide sequence set forth in SEQ ID NO: 2,
   N₁₀N₁₁N₁₂N₁₃GGN₁₄N₁₅N₁₆N₁₇N₁₈N₁₉N₂₀CCN₂₁N₂₂N₂₃ (SEQ ID NO: 2)
   (in SEQ ID NO: 2, N₁₄ to N₂₀ each independently represent any base, N₁₀ represents A or G, N₁₁ represents C or U, N₁₂ represents C or U, N₁₃ represents G or U, N₂₁ represents A or C, N₂₂ represents A or G, N₂₃ represents A or G, N₁₄ to N₂₀ form an anticodon loop, and N₁₁N₁₂N₁₃GG forms a base pair with CCN₂₁N₂₂N₂₃).
[5] The production method according to any one of [1] to [4], wherein the initiator tRNA further contains one nucleotide sequence selected from SEQ ID NOs: 3 to 6,
   GUCGG(N₂₄)ₘCCGGC (SEQ ID NO: 3)
   (in SEQ ID NO: 3, N₂₄'s each independently represent any base, m is an integer of 1 or more, (N₂₄)ₘ forms a T-loop, and GUCGG forms a T-stem with CCGGC)
   GUCGG(N₂₅)ₘCCGGU (SEQ ID NO: 4)
   (in SEQ ID NO: 4, N₂₅'s each independently represent any base, m is an integer of 1 or more, (N₂₅)ₘ forms a T-loop, and GUCGG forms a T-stem with CCGGU)
   GGCGG(N₂₆)ₘCCGCU (SEQ ID NO: 5)
   (in SEQ ID NO: 5, N₂₆'s each independently represent any base, m is an integer of 1 or more, (N₂₆)ₘ forms a T-loop, and GGCGG forms a T-stem with CCGCU)
   GGAGG(N₂₇)ₘCCUCU (SEQ ID NO: 6)
   (in SEQ ID NO: 6, N₂₇'s each independently represent any base, m is an integer of 1 or more, (N₂₇)ₘ forms a T-loop, and GGAGG forms a T-stem with CCUCU).
[6] The production method according to any one of [1] to [5], wherein the initiator tRNA further contains a nucleotide sequence set forth in SEQ ID NO: 7,
   N₂₈GN₂₉UC (SEQ ID NO: 7)
   (in SEQ ID NO: 7, N₂₈ represents A or G, N₂₉ represents A or G, and N₂₈GN₂₉UC forms a variable loop).
   [6-1]
      The production method according to any one of [1] to [6], in which a base at a 5' end of the initiator tRNA is mismatched with a base paired therewith.
   [6-2]
      The production method according to any one of [1] to [6-1], in which a base at a 5' end of the initiator tRNA is G and a base paired therewith is A.
   [6-3]
      The production method according to any one of [1] to [6-2], in which in the initiator tRNA, a base at a first position from a 5' end is mismatched with a base at a fifth position from a 3' end.
   [6-4]
      The production method according to any one of [1] to [6-3], in which in the initiator tRNA, a base at a first position from a 5' end is G and a base at a fifth position from a 3' end is A.
   [6-5]
      The production method according to any one of [1] to [6-4], wherein the initiator tRNA further contains a nucleotide sequence set forth in SEQ ID NO: 243,
   GGN₃₀N₃₁GN₃₂N₃₃ (SEQ ID NO: 243)
   (in SEQ ID NO: 243, N₃₀ represents C or G, N₃₁ represents U or G, N₃₂ represents A or G, N₃₃ represents U or G, the G at the first position does not form a base pair with a base paired therewith, each base of GN₃₀N₃₁GN₃₂N₃₃ forms a base pair with any of A, U, G, or C to form an acceptor stem, and it is preferable that the G at the first position in SEQ ID NO: 243 be a 5' end of the initiator tRNA of the present embodiment).
[7] The initiator tRNA according to any one of [1] to [6] (comprising [6-1] to [6-5]).
[8] A translation system comprising the initiator tRNA according to [7].
[9] A method for producing a peptide library, comprising a step of translating in a cell-free translation system using the initiator tRNA according to [7] or the translation system according to [8].
[10] A method for producing a peptide-mRNA complex library, which is a library of a complex of a peptide with an mRNA encoding the peptide, the method comprising a step of translating in a cell-free translation system using the initiator tRNA according to [7] or the translation system according to [8].
[11] A peptide library produced by the method according to [9].
[12] A peptide-mRNA complex library produced by the method according to [10].
[13] The peptide library according to [11], wherein the peptide library contains a peptide comprising, at an N terminus, a D-amino acid, a β-amino acid, a γ-amino acid, or L-proline, each comprising a nitrogen atom modified at an α-position, a β-position, or a γ-position.
[14] The peptide-mRNA complex library according to [12], wherein the peptide-mRNA complex library contains a complex of a peptide comprising, at an N terminus, a D-amino acid, a β-amino acid, a γ-amino acid, or L-proline, each comprising a nitrogen atom modified at an α-position, a β-position, or a γ-position, and an mRNA encoding the peptide.

### Advantageous Effect of Invention

According to the present invention, it is possible to provide a production method for a peptide, which enables various amino acids to be introduced efficiently and in a versatile manner at an N terminus of a peptide, and an initiator tRNA and a translation system for use in such a production method.

### Brief Description of Drawings

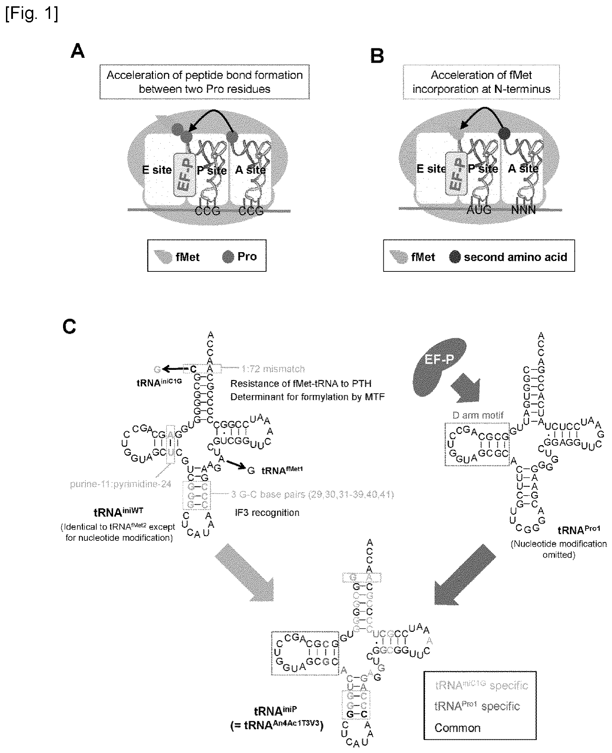

[Figure 1] Figure 1 is a schematic diagram illustrating EF-P recognizing a D-arm of a tRNA at a P site and promoting formation of a peptide bond. Figure 1A illustrates EF-P-mediated promotion of peptide bond formation between two consecutive Pro bases at the P site and the A site during translation elongation. EF-P recognizes a specific D-arm motif of peptidyl-prolyl-tRNA^{Pro} at the P site to promote the peptide bond formation. Figure 1B illustrates promotion by EF-P of peptide bond formation between an fMet-tRNA at the P site and an aminoacyl tRNA at the A site. This reaction occurs at translation initiation. Figure 1C illustrates the concept of developing a novel initiator tRNA that is efficiently recognized by EF-P to promote peptide bond formation. The nucleotide sequence of tRNA^{iniWT} is the same as the nucleotide sequence of E. coli tRNA^{fMet2}, except for the lack of nucleotide modification by in vitro transcription of tRNA^{iniWT}. The 5'-end C of tRNA^{inicC1G} is replaced with G. The D-arm motif of tRNA^{Pro1} is recognized by EF-P (indicated by a dark gray dotted line). By combining the structural features of tRNA^{iniWT} and tRNA^{Pro1}, tRNA^{iniP}, which is an artificial tRNA, has been devised. A nucleotide derived from tRNA^{iniC1G} is shown in gray, a nucleotide derived from tRNA^{Pro1} is shown in dark gray, and a common nucleotide is shown in black.

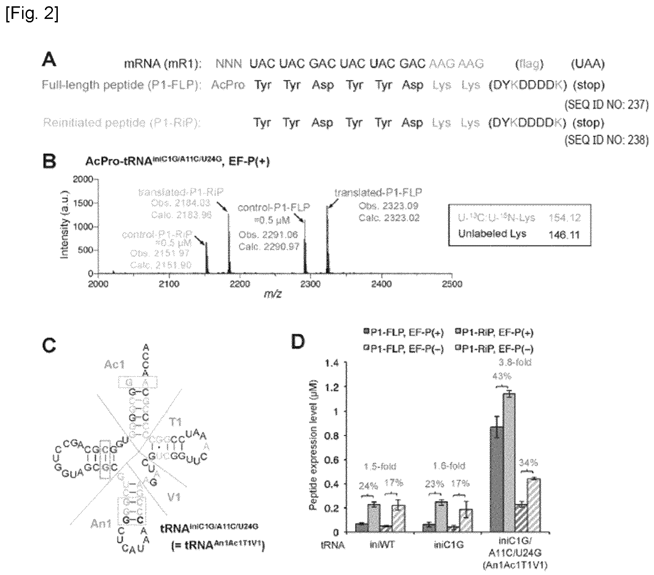

[Figure 2] Figure 2 illustrates transcription of a model peptide for evaluation of a tRNAⁱⁿⁱ mutant in the incorporation of AcPro at the N-terminus. Figure 2A illustrates a sequence of mRNA (mR1) and a corresponding peptide sequence P1. P1-FLP is a full-length peptide having N-acetyl-L-proline (AcPro) at the N-terminus. P1-RiP is a reinitiated peptide lacking AcPro. U-¹³C:U-¹⁵N-Lys is introduced into the translated P1-FLP and P1-RiP (two AAGs, two Lys residues, and two Ks in the flag). The mRNA sequence of the flag is GAC-TAC-AAG-GAC-GAC-GAC-GAC-AAG. Figure 2B illustrates MALDI-TOF MS of the transcription product. Translation has been performed using tRNA^{iniC1G/A11C/U24G} in the presence of 5 µM EF-P. The calculated value (calc.) and the measured value (obs.) of the m/z value of [M+H]⁺ are shown. The concentrations of the translated P1-FLP and P1-RiP have been determined by comparing with the peak intensities of internal controls P1-FLP and P1-RiP (0.5 µM). Figure 2C illustrates a secondary structure of tRNA^{iniC1G/A11C/U24G}. The anticodon stem, the acceptor stem, the T-stem, and the variable loop of the tRNA are referred to as An1, Ac1, T1, and V1, respectively. This tRNA is also referred to as tRNA^{An1Ac1T1V1}. Figure 2D illustrates the quantification of the expression levels of P1-FLP and P1-RiP. The translation of these peptides has been performed using tRNA^{iniWT}, tRNA^{iniC1G}, and tRNA^{An1Ac1T1V1} in the presence or absence of EF-P. The numbers above the bars indicate the increase ratio (fold) of the P1-FLP level to P1-FLP% and EF-P(+)/EF-P(-). The error bar represents S.D. (n = 3).

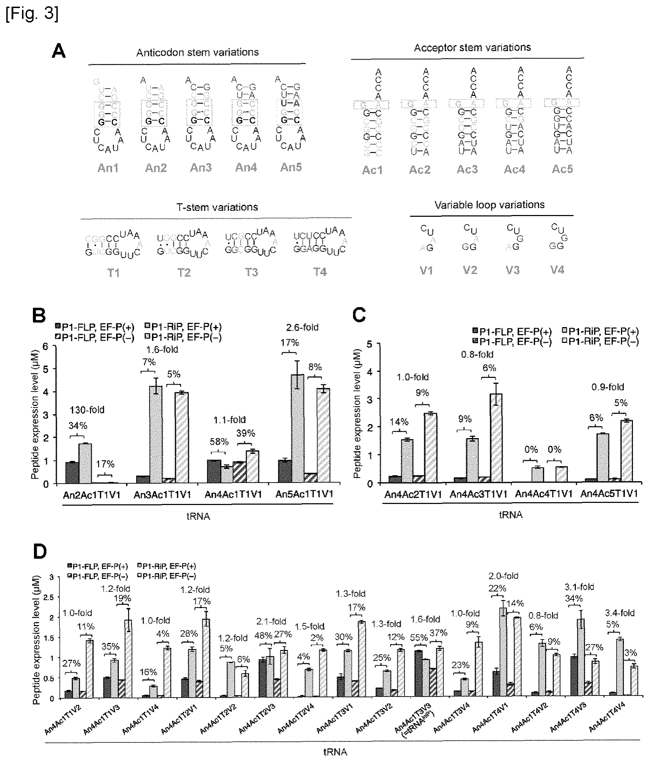

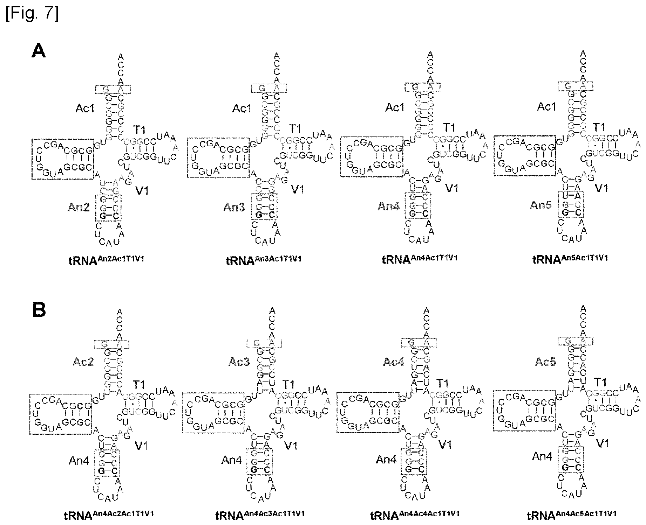

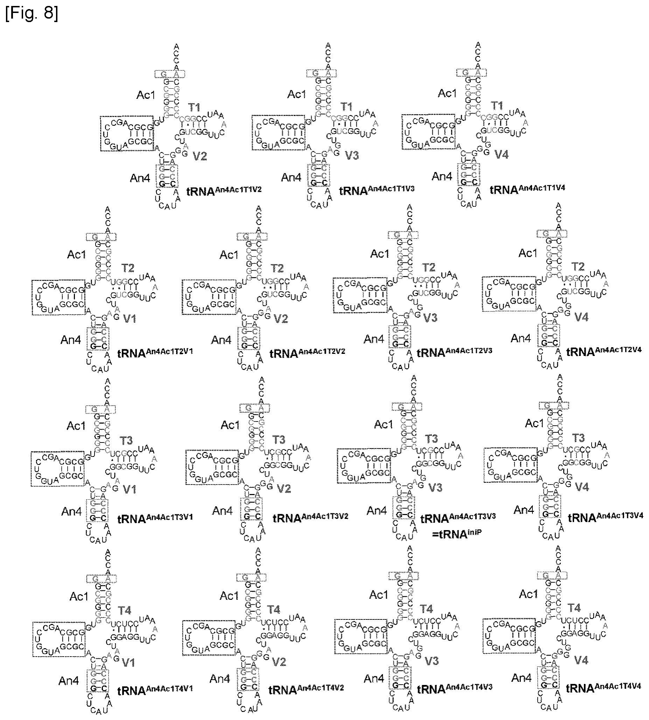

[Figure 3] Figure 3 illustrates the evaluation of tRNAⁱⁿⁱ having mutations in an anticodon stem, an acceptor stem, a T-stem, and a variable loop. Figure 3A illustrates structural variations of an anticodon stem, an acceptor stem, and a variable loop introduced into tRNAⁱⁿⁱ. Figures 3B to 3D illustrate the quantification of the expression levels of P1-FLP and P1-RiP for (B) the mutation of the anticodon stem, (C) the mutation of the acceptor stem, and (D) the mutation of the T-stem/the variable loop. The translation of the peptide has been performed in the presence and absence of EF-P. The numbers above the bars indicate the increase ratio (fold) of the P1-FLP level to P1-FLP% and EF-P(+)/EF-P(-). The error bar represents S.D. (n = 3). The secondary structure of the tRNAⁱⁿⁱ mutant can also be referred to Figures 7 and 8.

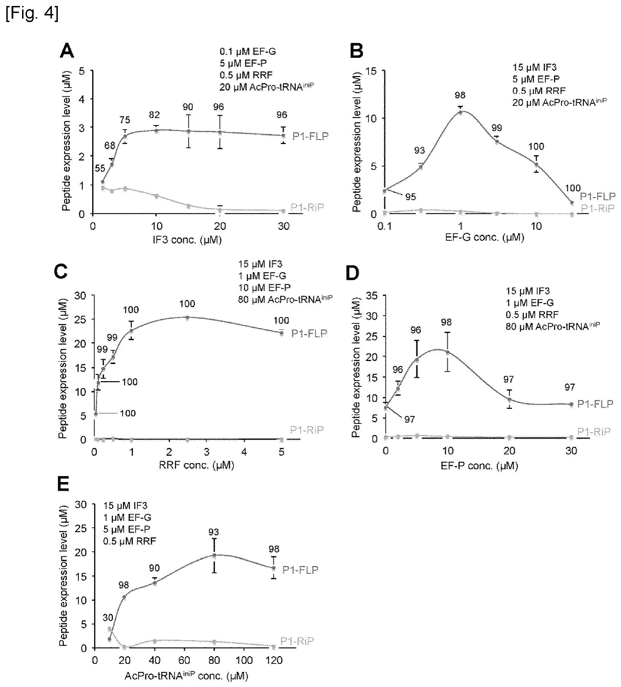

[Figure 4] Figure 4 illustrates the titration of the translation factor and AcPro-tRNA^{iniP} in the translation of the P1 peptide. Figure 4A illustrates the titration of IF3. Figure 4B illustrates the titration of EF-G. Figure 4C illustrates the titration of RRF. Figure 4D illustrates the titration of EF-P. Figure 4E illustrates an AcPro-tRNA^{iniP}. The dark gray and gray dots indicate the expression levels of P1-FLP and P1-RiP, respectively. The number above a dark gray dot indicates P1-FLP%. The error bar represents S.D. (n = 3).

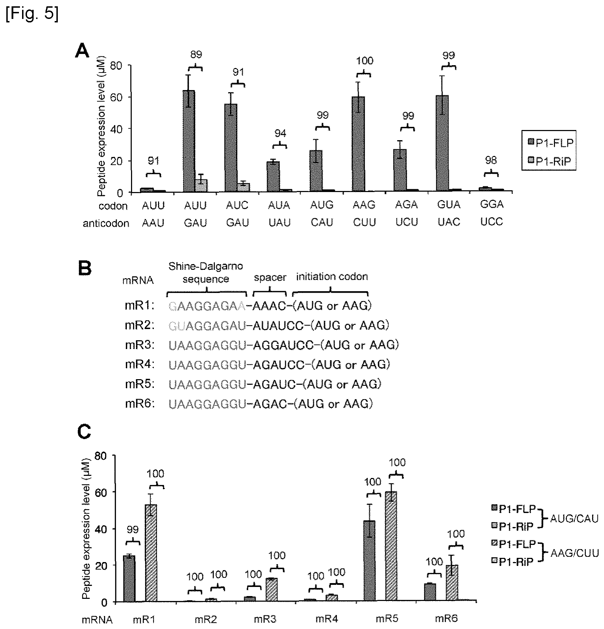

[Figure 5] Figure 5 illustrates optimization of a combination of a codon and an anticodon. Figure 5A illustrates the expression levels of P1-FLP and P1-RiP translated from mR1. Here, the initiation codon used for the incorporation of AcPro has been changed as shown in the lower part of the horizontal axis of the graph. 15 µM IF3, 1 µM EF-G, 2.5 µM RRF, 10 µM EF-P, and 160 µM AcPro-tRNA^{iniP} have been used. The anticodon of the tRNA^{iniP} has been similarly changed as shown in the lower part of the horizontal axis of the graph. The number above the bar indicates P1-FLP%. The error bar represents S.D. (n = 3). Figure 5B illustrates mR1 to mR6 which are mRNAs. The Shine-Dalgarno (SD) sequence and the spacer between the SD and the initiation codon have been optimized. A nucleotide complementary to the anti-SD of the 16S rRNA is shown in dark gray, and a non-complementary nucleotide is shown in gray. AUG or AAG was used as an initiation codon. Figure 5C illustrates the expression levels of P1-FLP and P1-RiP translated from mR1 to mR6. 15 µM IF3, 1 µM EF-G, 2.5 µM RRF, 10 µM EF-P, and 160 µM AcPro-tRNA^{iniP} have been used. The number above the bar indicates P1-FLP%. The error bar represents S.D. (n = 3).

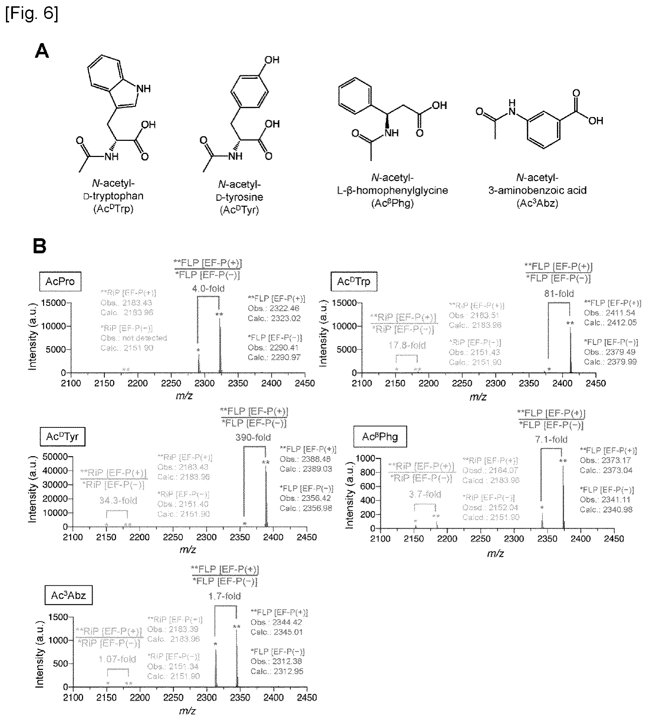

[Figure 6] Figure 6 illustrates that EF-P, in combination with tRNA^{iniP}, enhances the ribosome incorporation of the D-amino acid, the β-amino acid, and the γ-amino acid at the N-terminus. Figure 6A illustrates the structure of a non-proteinogenic amino acid that has been tested for ribosome incorporation at the N terminus. Ac^{D}Trp and Ac^{D}Tyr are representative substances of D-amino acids, and Ac^{β}Phg and Ac³Abz are each representative substances of β-amino acids and γ-amino acids, respectively. Figure 6B illustrates MALDI-TOF MS of the transcription product. The translation has been performed using mR5-AAG and aminoacyl-tRNA^{iniP}_{CUU} in the presence and absence of EF-P. 15 µM IF3, 1 µM EF-G, 2.5 µM RRF, 10 µM or 0 µM EF-P, and 160 µM aminoacyl-tRNA^{iniP}_{CUU} have been used. For translation in the presence of EF-P, the peptide has been labeled with U-¹³C:U-¹⁵N-Lys, and the unlabeled Lys has been incorporated for EF-P(-) translation. The EF-P(+) and EF-P(-) translation solutions have been mixed together and analyzed by MALDI-TOF MS. The calculated value (calc.) and the measured value (obs.) of the m/z value of [M+H]⁺ are shown. The increase ratio of the P1-FLP level to EF-P(+)/EF-P(-) was estimated based on the relative peak intensity.
[Figure 7] Figure 7 illustrates a secondary structure of a tRNAⁱⁿⁱ mutant. Figure 7A illustrates an anticodon stem mutant. Figure 7B illustrates an acceptor stem mutant.
[Figure 8] Figure 8 illustrates a secondary structure of a tRNAⁱⁿⁱ mutant (T-stem/variable loop mutant).

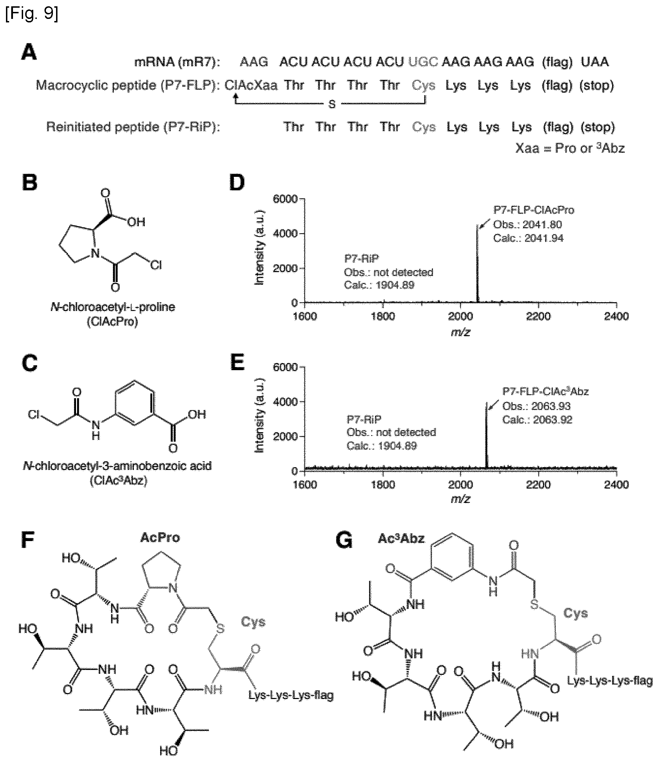

[Figure 9] Figure 9 illustrates the incorporation of N-chloroacetyl-L-proline or N-chloroacetyl-3-aminobenzoic acid for cyclization of a peptide at an N-terminus of the peptide. Figure 9A illustrates the sequence of the mRNA (mR7) used and the sequence of the peptide (P7) corresponding thereto. P7-FLP is a full-length cyclic peptide and P7-RiP is a reinitiated peptide lacking an N-terminal chloroacetyl amino acid (ClAcXaa). The flags of the mRNA and the peptide are GAC-UAC-AAG-GAC-GAC-GAC-GAC-AAG and Asp-Tyr-Lys-Asp-Asp-Asp-Asp-Lys, respectively. Figures 9B and 9C illustrate the structures of the introduced CIAcPro and CIAc³Abz, respectively. Figures 9D and 9E illustrate MALDI-TOF MS of the translated peptides. The translation has been performed using tRNA^{iniP}_{CUU}. The calculated value (calc.) and the measured value (obs.) of the m/z value of [M+H]⁺ are shown. Figures 9F and 9G illustrate chemical structures of a cyclic peptide P7-FLP comprising AcPro or Ac³Abz.

### Description of Embodiments

Hereinafter, embodiments for implementing the present invention will be described in detail. The present invention is not limited to the following embodiments and can be implemented with various modifications within the scope of the present invention.

The production method of the present embodiment is a production method for a peptide, comprising translating in a cell-free translation system using an initiator tRNA comprising a nucleotide sequence set forth in SEQ ID NO: 1.
GCGCN₁N₂N₃N₄N₅N₆N₇N₈N₉GCGC (SEQ ID NO: 1)
In SEQ ID NO: 1, N₁ to N₉ each independently represent any base, N₁ to N₉ form a D-loop, and GCGC forms base pairs with GCGC.
In SEQ ID NO: 1, the statement that GCGC forms base pairs with GCGC may be alternatively expressed as that 5'-GCGC-3' at positions 1 to 4 in SEQ ID NO: 1 forms base pairs with 3'-CGCG-5' at positions 17 to 14.

The present inventors have focused on a translation factor EF-P (Elongation Factor P) that promotes peptide bond formation in a peptide elongation reaction, regarding that in the production of a peptide by a cell-free translation system, the amino acid that can be introduced at the N-terminus of the peptide is limited due to the N-terminal drop-off-reinitiation. EF-P plays an important role in promoting the elongation of proline-proline (Pro-Pro) bond formation, and alleviates translation elongation stalling (ribosome stalling) caused by the drop-off of peptidyl-tRNA in a Pro-Pro sequence (Figure 1A). The present inventors have hypothesized that EF-P functions not only in the elongation reaction but also in the initiation reaction (Figure 1B), and that the N-terminal drop-off-reinitiation can also be suppressed by EF-P. Furthermore, the present inventors have considered that, in a case where the EF-P can suppress the N-terminal drop-off-reinitiation, it is possible to efficiently synthesize, in the presence of EF-P, an exogenous peptide into which various amino acids comprising L-proline and non-proteinogenic amino acids such as D-amino acids, β-amino acids, and γ-amino acids are introduced at the N-terminus.

EF-P recognizes the specific D-arm of tRNA^{Pro1}, tRNA^{Pro2}, and tRNA^{Pro3} which are tRNA^{Pro} isoacceptors to promote the peptidyl transfer of Pro. The inventors of the present invention have expected that the peptidyl transfer between an N-terminal amino acid (an amino acid charged to the initiator tRNA) and a second amino acid is enhanced by replacing a D-arm of the initiator tRNA (tRNAⁱⁿⁱ) with a D-arm of the tRNA^{Pro} isoacceptor, and have invented a novel initiator tRNA in which a characteristic structures of a wild type (WT) tRNAⁱⁿⁱ and a tRNA^{Pro} isoacceptor are fused to efficiently incorporate various amino acids at the N-terminal.

According to the present invention, it is possible to provide a method capable of significantly increasing the number of types of amino acids that can be used for initiating translation and capable of producing a peptide comprising various N-terminal amino acids by producing a peptide by a cell-free translation system using a specific initiator tRNA described in detail later. That is, the present invention provides a highly versatile method for producing a peptide by a cell-free translation system.

In the present specification, the initiator tRNA means a tRNA that is used for initiation of translation of mRNA. The initiator tRNA may comprise an acceptor stem, a D-arm, an anticodon stem, an anticodon loop, a variable loop, and a T-arm. In the cell-free translation system, translation is initiated by translation initiation factor IF2 recognizing that the amino group of the amino acid (however, an imino group for a secondary amino acid such as proline) charged to the initiator tRNA is modified. In addition, not only in a case where a modified amino acid is charged to the initiator tRNA, but also in a case where a peptide is charged thereto, there is a possibility that the initiator tRNA is recognized by the translation initiation factor IF2 and translation is initiated. In addition, the methionyl tRNA transformylase that modifies the amino group of methionine charged to the initiator tRNA recognizes that the base at the 5'-end of the tRNA does not form a base pair with the base paired therewith (that is, mismatched), and performs formylation.

Therefore, the initiator tRNA may be a tRNA that is charged with an amino acid in which an amino group is modified (however, an imino group in the case of a secondary amino acid such as proline) or a tRNA that is charged with such an amino acid, and may be a tRNA in which a base at a 5'-end is mismatched with a base paired therewith.

In the amino acid charged to the initiator tRNA, a nitrogen atom of an amino group or an imino group is preferably modified and a nitrogen atom at an α-position, a β-position, or a γ-position is more preferably modified. More specifically, it is preferable that a nitrogen atom of an amino group is modified in a case of a primary amino acid and a nitrogen atom of an imino group is modified in a case of a secondary amino acid such as proline, and it is more preferable that a nitrogen atom of an amino group or an imino group at an α-position is modified in an α-amino acid, a nitrogen atom of an amino group or an imino group at a β-position is modified in a β-amino acid, and a nitrogen atom of an amino group or an imino group at a γ-position is modified in a γ-amino acid.

In the production method of the present embodiment, the initiator tRNA charged with the modified amino acid may be added to the cell-free translation system, or the initiator tRNA charged with the unmodified amino acid and a modifying enzyme such as methionyl-tRNA transferase may be added to the cell-free translation system to modify the amino acid in the system.

The modification is preferably a modification (acylation) with an acyl group which may have a substituent, and more preferably a modification (acetylation or formylation) with an acetyl group which may have a substituent, or a formyl group. Here, the substituent is not particularly limited, and examples thereof comprise an alkyl group (preferably a C1 to C3 alkyl group) and a halogen atom (preferably a chlorine atom).

In the present specification, a combination of mismatches of the first base of the initiator tRNA is not particularly limited. Examples of the combination of bases which is a mismatch comprise guanine (G) and adenine (A), G and uracil (U), A and cytosine (C), C and U, A and A, G and G, C and C, and U and U, and a combination of G and A is preferable. Therefore, it is preferable that the base at the 5'-end of the initiator tRNA of the present embodiment is G and the base paired therewith is A. In the wild-type initiator tRNA, since the base pair at position 72 typically corresponds to the base pair at position 1, the bases at position 1 and 72 are mismatched. Therefore, in the initiator tRNA of the present embodiment, it is preferable that in the initiator tRNA of the present embodiment, the base at a first position and the base corresponding to the base at a 72nd position of the wild-type initiator tRNA in a case of being aligned with the wild-type initiator tRNA are mismatched. Alternatively, in the initiator tRNA of the present embodiment, it is also preferable that the base at the first position from the 5'-end and the base at the fifth position from the 3'-end are mismatched, and it is more preferable that the base at the first position from the 5'-end is G or C (preferably G), the base at the fifth position from the 3'-end is A, and these bases are paired.

In the present specification, the terms "to charge" and "charged" are used synonymously with "to bind" and "bonded" or "to link" and "linked". Therefore, the "initiator tRNA that charges (is charged to) an amino acid" means a initiator tRNA in which an amino acid is acylated (has been acylated) to the initiator tRNA, and it is also synonymous with that an amino acid is "aminoacylated" to the initiator tRNA.

EF-P recognizes a nine-base D-loop having a structure closed by a stable D-stem sequence of four base pairs, which is a specific D-arm motif found in the tRNA^{Pro} isoacceptor.

In the present embodiment, the nucleotide sequence set forth in SEQ ID NO: 1 represents a nucleotide sequence of a D-arm consisting of a D-loop and a D-stem. The present inventors have found that, by introducing the nucleotide sequence set forth in SEQ ID NO: 1 into the initiator tRNA, peptidyl transfer can be promoted by EF-P, and various amino acids comprising proline and a non-proteinogenic amino acid can be efficiently introduced at the N-terminus of the peptide.

In the initiator tRNA comprising the nucleotide sequence set forth in SEQ ID NO: 1 of the present embodiment, other structures, that is, the acceptor stem, the anticodon stem, the anticodon loop, the variable loop, and the T-arm may have any the nucleotide sequences. Among these, the anticodon loop may appropriately have a nucleotide sequence corresponding to a codon to which an amino acid to be introduced by the initiator tRNA of the present embodiment is assigned. The 3'-end of the initiator tRNA has a CCA sequence and may be bonded to any amino acid.

It is preferable that the nucleotide sequence of N₁ to N₉ in SEQ ID NO: 1 is a nucleotide sequence set forth in SEQ ID NO: 8.
AGCCUGGUA (SEQ ID NO: 8)

The nucleotide sequence set forth in SEQ ID NO: 8 forms a D-loop in the initiator tRNA.

In SEQ ID NO: 8, one or more bases may be substituted, deleted, or inserted.

The fact that a plurality of bases are substituted means that, in the 9 bases forming the D-loop of the nucleotide sequence set forth in SEQ ID NO: 8, 2, 3, 4, 5, 6, 7, 8, or 9 bases may be substituted, and 1 to 4 bases may be substituted, 1 to 3 bases may be substituted, 1 or 2 bases may be substituted, or 1 base may be substituted.

The fact that a plurality of bases are deleted means that, in the 9 bases forming the D-loop of the nucleotide sequence set forth in SEQ ID NO: 8, 2, 3, 4, 5, 6, 7, 8, or 9 bases may be deleted, and 1 to 4 bases may be deleted, 1 to 3 bases may be deleted, 1 or 2 bases may be deleted, or 1 base may be deleted.

In a case where a base is inserted, in the 9 bases forming the D-loop of the nucleotide sequence set forth in SEQ ID NO: 8, 1 to 6 bases may be inserted, 1 to 5 bases may be inserted, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NO: 8, the nucleotide sequence may have a homology of 70% or more, 80% or more, 85% or more, 90% or more, or 95% or more with the nucleotide sequence set forth in SEQ ID NO: 8.

The nucleotide sequence set forth in SEQ ID NO: 1 is preferably a nucleotide sequence set forth in SEQ ID NO: 9.
GCGCAGCCUGGUAGCGC (SEQ ID NO: 9)

The nucleotide sequence set forth in SEQ ID NO: 9 forms a D-arm consisting of a D-loop and a D-stem in the initiator tRNA.

In SEQ ID NO: 9, one or more bases may be substituted in a portion other than GCGC at positions 1 to 4 and GCGC at positions 14 to 17.

The fact that a plurality of bases are substituted means that, in the 9 bases forming the D-loop of the nucleotide sequence set forth in SEQ ID NO: 9, 2, 3, 4, 5, 6, 7, 8, 9, 10, 11, 12, 13, 14, 15, 16, or 17 bases may be substituted, and 1 to 4 bases may be substituted, 1 to 3 bases may be substituted, 1 or 2 bases may be substituted, or 1 base may be substituted.

In addition, in a case where one or more bases are substituted in the nucleotide sequence set forth in SEQ ID NO: 9, the nucleotide sequence may have a homology of 70% or more, 80% or more, 85% or more, 90% or more, or 95% or more with the nucleotide sequence set forth in SEQ ID NO: 9.

The initiator tRNA of the present embodiment is preferably an initiator tRNA further comprises a nucleotide sequence set forth in SEQ ID NO: 2.
N₁₀N₁₁N₁₂N₁₃GGN₁₄N₁₅N₁₆N₁₇N₁₈N₁₉N₂₀CCN₂₁N₂₂N₂₃ (SEQ ID NO: 2)

In SEQ ID NO: 2, N₁₄ to N₂₀ each independently represent any base, N₁₀ represents A or G, N₁₁ represents C or U, N₁₂ represents C or U, N₁₃ represents G or U, N₂₁ represents A or C, N₂₂ represents A or G, N₂₃ represents A or G, N₁₄ to N₂₀ form an anticodon loop, and N₁₁N₁₂N₁₃GG forms a base pair with CCN₂₁N₂₂N₂₃.

In SEQ ID NO: 2, the statement that N₁₁N₁₂N₁₃GG forms base pairs with CCN₂₁N₂₂N₂₃ may be alternatively expressed as that 5'-N₁₁N₁₂N₁₃GG-3' at positions 2 to 6 in SEQ ID NO: 2 forms base pairs with 3'-N₂₃N₂₂N₂₁CC-5' at positions 18 to 14.

In the present embodiment, the nucleotide sequence set forth in SEQ ID NO: 2 is a nucleotide sequence of a portion consisting of an anticodon stem and an anticodon loop, and by introducing the nucleotide sequence set forth in SEQ ID NO: 2 into the initiator tRNA, peptidyl transfer by EF-P can be promoted, and various amino acids comprising proline and a non-proteinogenic amino acid can be more efficiently introduced at the N-terminus of the peptide.

The SEQ ID NO: 2 is preferably represented by SEQ ID NO: 10 or 11.
N₁₆N₁₁N₁₂GGGN₁₄N₁₅N₁₆N₁₇N₁₃N₁₉N₂₆CCCN₂₂N₂₃ (SEQ ID NO: 10)
N₁₆N₁₁N₁₂UGGN₁₄N₁₅N₁₆N₁₇N₁₃N₁₉N₂₆CCAN₂₂N₂₃ (SEQ ID NO: 11)

The nucleotide sequences set forth in SEQ ID NOs: 10 and 11 form an anticodon stem and an anticodon loop.

The SEQ ID NO: 2 is more preferably represented by any of SEQ ID NOs: 12 to 16, and still more preferably represented by SEQ ID NO: 15.
GUCGGGN₁₄N₁₅N₁₆N₁₇N₁₈N₁₉N₂₀CCCGA (SEQ ID NO: 12)
AUCGGGN₁₄N₁₅N₁₆N₁₇N₁₈N₁₉N₂₀CCCGA (SEQ ID NO: 13)
ACCGGGN₁₄N₁₅N₁₆N₁₇N₁₈N₁₉N₂₀CCCGG (SEQ ID NO: 14)
ACUGGGN₁₄N₁₅N₁₆N₁₇N₁₈N₁₉N₂₀CCCAG (SEQ ID NO: 15)
ACUUGGN₁₄N₁₅N₁₆N₁₇N₁₈N₁₉N₂₀CCAAG (SEQ ID NO: 16)

The nucleotide sequences set forth in SEQ ID NOs: 12 to 16 form an anticodon stem and an anticodon loop.

SEQ ID NO: 2 is also preferably represented by SEQ ID NO: 17.
N₁₀N₁₁N₁₂N₁₃GGCUN₁₆N₁₇N₁₈AACCN₂₁N₂₂N₂₃ (SEQ ID NO: 17)

The nucleotide sequence set forth in SEQ ID NO: 17 forms an anticodon stem and an anticodon loop.

The nucleotide sequences of N₁₆ to N₁₈ in SEQ ID NO: 2 and 10 to 17 form an anticodon.

The anticodon of the initiator tRNA has a nucleotide sequence complementary to the initiation codon. In nature, since the initiator tRNA always encodes methionine (formylmethionine), the initiator tRNA has an anticodon corresponding to methionine. In this case, AUG, which is a methionine codon, is generally used as the initiation codon.

On the other hand, in the present embodiment, the initiation codon is not limited to AUG, and any codon comprising all the codons described in the "natural genetic code table" shown in Table 1 described later can be used as the initiation codon. Therefore, since the tRNA of the present embodiment can have a nucleotide sequence complementary to any initiation codon as an anticodon, the nucleotide sequences of N₁₆ to N₁₈ in SEQ ID NO: 2 and 10 to 17 are arbitrary.

In the present specification, the complementary nucleotide sequence is not limited to a nucleotide sequence in which each base forms a Watson-Crick base pair, and also comprises a nucleotide sequence in which a wobble base pair is formed. The wobble base pair means a base pair in which a hydrogen bond is formed between G and U, between inosine (I) and U, between I and A, and between I and C. In addition, the complementary nucleotide sequence may not have 100% complementarity with the target nucleotide sequence, and for example, 1 to 3, 1 or 2, or 1 non-complementary bases with respect to the target nucleotide sequence may be comprised.

In the present embodiment, the combination of the initiation codon:anticodon is preferably AUU:AAU, AUU:GAU, AUC:GAU, AUG:CAU, AAG:CUU, AGA:UCU, GUA:UAC, or GGA:UCC, and more preferably AAG:CUU, GUA:UAC, or AUG:CAU. The initiator tRNA preferably has any anticodon of the above-described combinations as a nucleotide sequence of N₁₆ to N₁₈.

The SEQ ID NO: 2 is preferably represented by any of SEQ ID NOs: 18 to 22, and more preferably represented by SEQ ID NO: 21.
GUCGGGCUNNNAACCCGA (SEQ ID NO: 18)
AUCGGGCUNNNAACCCGA (SEQ ID NO: 19)
ACCGGGCUNNNAACCCGG (SEQ ID NO: 20)
ACUGGGCUNNNAACCCAG (SEQ ID NO: 21)
ACUUGGCUNNNAACCAAG (SEQ ID NO: 22)

The nucleotide sequences set forth in SEQ ID NOs: 18 to 22 form an anticodon stem and an anticodon loop, and NNN represents any anticodon. NNN may be the above-described anticodon as a preferred combination of initiation codon: anticodon.

In SEQ ID NOs: 2 and 10 to 22, one or more bases may be substituted, deleted, or inserted.

The fact that a plurality of bases are substituted means that, in the 18 bases forming the anticodon stem and anticodon loop of the nucleotide sequence set forth in SEQ ID NO: 2 and 10 to 22, 2, 3, 4, 5, 6, 7, 8, 9, 10, 11, 12, 13, 14, 15, 16, 17, or 18 bases may be substituted, and 1 to 11 bases may be substituted, 1 to 10 bases may be substituted, 1 to 9 bases may be substituted, 1 to 8 bases may be substituted, 1 to 7 bases may be substituted, 1 to 6 bases may be substituted, 1 to 5 bases may be substituted, 1 to 4 bases may be substituted, 1 to 3 bases may be substituted, 1 or 2 bases may be substituted, or 1 base may be substituted.

The fact that a plurality of bases are deleted means that, in the 18 bases forming the anticodon stem and anticodon loop of the nucleotide sequence set forth in SEQ ID NO: 2 and 10 to 22, 2, 3, 4, 5, 6, 7, 8, 9, 10, 11, 12, 13, 14, 15, 16, 17, or 18 bases may be deleted, and 1 to 11 bases may be deleted, 1 to 10 bases may be deleted, 1 to 9 bases may be deleted, 1 to 8 bases may be deleted, 1 to 7 bases may be deleted, 1 to 6 bases may be deleted, 1 to 5 bases may be deleted, 1 to 4 bases may be deleted, 1 to 3 bases may be deleted, 1 or 2 bases may be deleted, or 1 base may be deleted.

In a case where a base is inserted, in 18 bases forming an anticodon stem and an anticodon loop of the nucleotide sequences set forth in SEQ ID NOs: 2 and 10 to 22, 1 to 11 bases may be inserted, 1 to 10 bases may be inserted, 1 to 9 bases may be inserted, 1 to 8 bases may be inserted, 1 to 7 bases may be inserted, 1 to 6 bases may be inserted, 1 to 5 bases may be inserted, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NOs: 2 and 10 to 22, the nucleotide sequences may each have a homology of 70% or more, 80% or more, 85% or more, 90% or more, or 95% or more with the nucleotide sequences set forth in SEQ ID NOs: 2 and 10 to 22.

The initiator tRNA of the present embodiment is preferably an initiator tRNA further comprising one nucleotide sequence selected from SEQ ID NOs: 3 to 6.
GUCGG(N₂₄)ₘCCGGC (SEQ ID NO: 3)

In SEQ ID NO: 3, N₂₄'s each independently represent any base, m is an integer of 1 or more, (N₂₄)ₘ forms a T-loop, and GUCGG forms a T-stem with CCGGC.
GUCGG(N₂₅)ₘCCGGU (SEQ ID NO: 4)

In SEQ ID NO: 4, N₂₅'s each independently represent any base, m is an integer of 1 or more, (N₂₅)ₘ forms a T-loop, and GUCGG forms a T-stem with CCGGU.
GGCGG(N₂₆)ₘCCGCU (SEQ ID NO: 5)

In SEQ ID NO: 5, N₂₆'s each independently represent any base, m is an integer of 1 or more, (N₂₆)ₘ forms a T-loop, and GGCGG forms a T-stem with CCGCU.
GGAGG(N₂₇)ₘCCUCU (SEQ ID NO: 6)

In SEQ ID NO: 6, N₂₇'s each independently represent any base, m is an integer of 1 or more, (N₂₇)ₘ forms a T-loop, and GGAGG forms a T-stem with CCUCU.

In SEQ ID NO: 3, the statement that GUCGG forms a T-stem with CCGGC may be alternatively expressed as that 5'-GUCGG-3' forms base pairs with 3'-CGGCC-5' to form the T-stem. The same applies to SEQ ID NOs: 4 to 6. It is noted that in the T-stem, all bases do not necessarily form a base pair.

In the present embodiment, the nucleotide sequences set forth in SEQ ID NOs: 3 to 6 are each a T-arm consisting of a T-loop and a T-stem, and by introducing the nucleotide sequences set forth in SEQ ID NOs: 3 to 6 into the initiator tRNA, the peptidyl transfer by EF-P can be promoted, and various amino acids comprising proline and a non-proteinogenic amino acid can be more efficiently introduced at the N-terminus of the peptide.

In SEQ ID NOs: 3 to 6, m is not particularly limited as long as (N₂₄)ₘ, (N₂₅)ₘ, (N₂₆)ₘ, and (N₂₇)ₘ form a T-loop, but may be an integer of 2, 3, 4, 5, 6, 7, 8, 9, or 10, may be in any range having these numerical values as an upper limit value and a lower limit value, and is preferably an integer of 5 to 9 and more preferably an integer of 6 to 8.

(N₂₄)ₘ, (N₂₅)ₘ, (N₂₆)ₘ, and (N₂₇)ₘ are each preferably a nucleotide sequence set forth in SEQ ID NO: 23, which is derived from the T-loop of the tRNA^{iniWT} described in Examples.
UUCAAAU (SEQ ID NO: 23)

The nucleotide sequence set forth in SEQ ID NO: 23 forms a T-loop in the initiator tRNA.

In SEQ ID NO: 23, one or more bases may be substituted, deleted, or inserted.

The fact that a plurality of bases are substituted means that, in the 7 bases forming the T-loop of the nucleotide sequence set forth in SEQ ID NO: 23, 2, 3, 4, 5, 6, or 7 bases may be substituted, and 1 to 4 bases may be substituted, 1 to 3 bases may be substituted, 1 or 2 bases may be substituted, or 1 base may be substituted.

The fact that a plurality of bases are deleted means that, in the 7 bases forming the T-loop of the nucleotide sequence set forth in SEQ ID NO: 23, 2, 3, 4, 5, 6, or 7 bases may be deleted, and 1 to 4 bases may be deleted, 1 to 3 bases may be deleted, 1 or 2 bases may be deleted, or 1 base may be deleted.

In a case where a base is inserted, in the 7 bases forming the T-loop of the nucleotide sequence set forth in SEQ ID NO: 23, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NO: 23, the nucleotide sequence may have a homology of 70% or more, 80% or more, 85% or more, 90% or more, or 95% or more with the nucleotide sequence set forth in SEQ ID NO: 23.

It is preferable that SEQ ID NOs: 3 to 6 are each represented by SEQ ID NOs: 24 to 27. In addition, the initiator tRNA of the present embodiment preferably has a nucleotide sequence set forth in SEQ ID NO: 26.
GUCGGUUCAAAUCCGGC (SEQ ID NO: 24)
GUCGGUUCAAAUCCGGU (SEQ ID NO: 25)
GGCGGUUCAAAUCCGCU (SEQ ID NO: 26)
GGAGGUUCAAAUCCUCU (SEQ ID NO: 27)

In the nucleotide sequences set forth in SEQ ID NOs: 3 to 6 and 24 to 27, one or more bases may be substituted, deleted, or inserted, and preferably, one or more bases may be substituted, deleted, or inserted in the nucleotide sequence constituting the T-loop.

The fact that a plurality of bases are substituted means that, in the 17 bases forming the T-arm of the nucleotide sequence set forth in SEQ ID NOs: 3 to 6 and 24 to 27, 1, 2, 3, 4, 5, 6, 7, 8, 9, 10, 11, 12, 13, 14, 15, 16, or 17 bases may be substituted, and 1 to 11 bases may be substituted, 1 to 10 bases may be substituted, 1 to 9 bases may be substituted, 1 to 8 bases may be substituted, 1 to 7 bases may be substituted, 1 to 6 bases may be substituted, 1 to 5 bases may be substituted, 1 to 4 bases may be substituted, 1 to 3 bases may be substituted, 1 or 2 bases may be substituted, or 1 base may be substituted.

The fact that a plurality of bases are deleted means that, in the 17 bases forming the T-arm of the nucleotide sequence set forth in SEQ ID NOs: 3 to 6 and 24 to 27, 1, 2, 3, 4, 5, 6, 7, 8, 9, 10, 11, 12, 13, 14, 15, 16, or 17 bases may be deleted, and 1 to 11 bases may be deleted, 1 to 10 bases may be deleted, 1 to 9 bases may be deleted, 1 to 8 bases may be deleted, 1 to 7 bases may be deleted, 1 to 6 bases may be deleted, 1 to 5 bases may be deleted, 1 to 4 bases may be deleted, 1 to 3 bases may be deleted, 1 or 2 bases may be deleted, or 1 base may be deleted.

In a case where a base is inserted, in the 17 bases forming the T-arm of the nucleotide sequence set forth in SEQ ID NOs: 3 to 6 and 24 to 27, 1 to 11 bases may be inserted, 1 to 10 bases may be inserted, 1 to 9 bases may be inserted, 1 to 8 bases may be inserted, 1 to 7 bases may be inserted, 1 to 6 bases may be inserted, 1 to 5 bases may be inserted, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NOs: 3 to 6 and 24 to 27, the nucleotide sequences may each have a homology of 70% or more, 80% or more, 85% or more, 90% or more, or 95% or more with the nucleotide sequences set forth in SEQ ID NOs: 3 to 6 and 24 to 27.

The initiator tRNA of the present embodiment is preferably an initiator tRNA further comprises a nucleotide sequence set forth in SEQ ID NO: 7.
N₂₈GN₂₉UC (SEQ ID NO: 7)

In SEQ ID NO: 7, N₂₈ represents A or G, N₂₉ represents A or G, and N₂₈GN₂₉UC forms a variable loop.

In the present embodiment, the nucleotide sequence set forth in SEQ ID NO: 7 is a variable loop. By introducing the nucleotide sequence set forth in SEQ ID NO: 7 into the initiator tRNA, peptidyl transfer by EF-P can be promoted, and various amino acids comprising proline and a non-proteinogenic amino acid can be more efficiently introduced at the N-terminus of the peptide.

The SEQ ID NO: 7 is preferably represented by any of SEQ ID NOs: 28 to 31, and more preferably represented by SEQ ID NO: 30.
AGAUC (SEQ ID NO: 28)
GGAUC (SEQ ID NO: 29)
AGGUC (SEQ ID NO: 30)
GGGUC (SEQ ID NO: 31)

The nucleotide sequences set forth in SEQ ID NOs: 28 to 31 each form a variable loop.

In SEQ ID NOs: 7 and 28 to 31, one or more bases may be substituted, deleted, or inserted.

The fact that a plurality of bases are substituted means that, in the 5 bases forming the variable loop of the nucleotide sequence set forth in SEQ ID NOs: 7 and 28 to 31, 2, 3, 4, or 5 bases may be substituted, and 1 to 4 bases may be substituted, 1 to 3 bases may be substituted, 1 or 2 bases may be substituted, or 1 base may be substituted.

The fact that a plurality of bases are deleted means that, in the 5 bases forming the variable loop of the nucleotide sequence set forth in SEQ ID NOs: 7 and 28 to 31, 2, 3, 4 or 5 bases may be deleted, and 1 to 4 bases may be deleted, 1 to 3 bases may be deleted, 1 or 2 bases may be deleted, or 1 base may be deleted.

In a case where a base is inserted, in the 5 bases forming the variable loop of the nucleotide sequence set forth in SEQ ID NOs: 7 and 28 to 31, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NOs: 7 and 28 to 31, the nucleotide sequences may each have a homology of 70% or more, 80% or more, 85% or more, 90% or more, or 95% or more with the nucleotide sequences set forth in SEQ ID NOs: 7 and 28 to 31.

The initiator tRNA of the present embodiment is preferably an initiator tRNA further comprises a nucleotide sequence set forth in SEQ ID NO: 243.
GGN₃₀N₃₁GN₃₂N₃₃ (SEQ ID NO: 243)

In SEQ ID NO: 243, N₃₀ represents C or G, N₃₁ represents U or G, N₃₂ represents A or G, N₃₃ represents U or G, the G at the first position does not form a base pair with a base paired therewith, and each base of GN₃₀N₃₁GN₃₂N₃₃ forms a base pair with any of A, U, G, or C to form an acceptor stem. It is preferable that G at the first position in SEQ ID NO: 243 be the 5'-end of the initiator tRNA of the present embodiment.

The SEQ ID NO: 243 is preferably represented by any of SEQ ID NOs: 244 to 248, and more preferably represented by SEQ ID NO: 244.
GGCGGGG (SEQ ID NO: 244)
GGCGGGU (SEQ ID NO: 245)
GGCGGAU (SEQ ID NO: 246)
GGCUGAU (SEQ ID NO: 247)
GGGUGAU (SEQ ID NO: 248)

The nucleotide sequences set forth in SEQ ID NOs: 244 to 248 form an acceptor stem together with base pairs paired therewith. It is preferable that G at the first position in SEQ ID NOs: 244 to 248 does not form a base pair with a base paired therewith.

In SEQ ID NOs: 243 to 248, one or more bases may be substituted, deleted, or inserted.

The fact that a plurality of bases are substituted means that, in the 7 bases forming the nucleotide sequence set forth in SEQ ID NOs: 243-248, 2, 3, 4, 5, 6, or 7 bases may be substituted, and 1 to 4 bases may be substituted, 1 to 3 bases may be substituted, 1 or 2 bases may be substituted, or 1 base may be substituted.

The fact that a plurality of bases are deleted means that, in the 7 bases forming the nucleotide sequence set forth in SEQ ID NOs: 243-248, 2, 3, 4, 5, 6, or 7 bases may be deleted, and 1 to 4 bases may be deleted, 1 to 3 bases may be deleted, 1 or 2 bases may be deleted, or 1 base may be deleted.

In a case where a base is inserted, in the 7 bases forming the nucleotide sequence set forth in SEQ ID NOs: 243-248, 1 to 4 bases may be inserted, 1 to 3 bases may be inserted, 1 or 2 bases may be inserted, or 1 base may be inserted.

In addition, in a case where one or more bases are substituted, deleted, or inserted in the nucleotide sequence set forth in SEQ ID NOs: 243-248, the nucleotide sequences may each have a homology of 70% or more, 80% or more, 85% or more, 90% or more, or 95% or more with the nucleotide sequences set forth in SEQ ID NOs: 243-248.

From the viewpoint of promoting peptidyl transfer by EF-P, the suitable initiator tRNA used in the production method of the present embodiment preferably comprises, in addition to the nucleotide sequence set forth in SEQ ID NO: 1, any of the nucleotide sequence set forth in SEQ ID NO: 2, one nucleotide sequence selected from SEQ ID NOs: 3 to 6, the nucleotide sequence set forth in SEQ ID NO: 7, or the nucleotide sequence set forth in SEQ ID NO: 243, and more preferably comprises, in addition to the nucleotide sequence set forth in SEQ ID NO: 1, all of the nucleotide sequence set forth in SEQ ID NO: 2, one nucleotide sequence selected from SEQ ID NOs: 3 to 6, the nucleotide sequence set forth in SEQ ID NO: 7, and the nucleotide sequence set forth in SEQ ID NO: 243. The preferred aspects of the nucleotide sequence set forth in SEQ ID NO: 2, the nucleotide sequences set forth in SEQ ID NOs: 3 to 6, the nucleotide sequence set forth in SEQ ID NO: 7, and the nucleotide sequence set forth in SEQ ID NO: 243 are as described above, and preferred nucleotide sequences, more preferred nucleotide sequences, particularly preferred nucleotide sequences, as described for each, can be optionally combined.

In the present specification, a suitable initiator tRNA used in the production method according to the present embodiment is referred to as a tRNA^{iniP}. The initiator tRNA of the present embodiment may be any initiator tRNA represented by tRNA^{iniP} described in Examples.

In the initiator tRNA used in the production method of the present embodiment, a nucleotide sequence other than SEQ ID NO: 1 may be a sequence derived from a wild-type tRNA, a sequence derived from a wild-type tRNA derived from Escherichia coli, a sequence derived from a wild-type initiator tRNA derived from Escherichia coli, or a sequence derived from an artificial tRNA prepared by in vitro transcription.

In the initiator tRNA used in the production method of the present embodiment, the above-described aspects may be optionally combined for each of the acceptor stem, the D-arm, the anticodon stem, the anticodon loop, the variable loop, and the T-arm.

In the production method of the present embodiment, a peptide comprising the charged amino acid at the N-terminus is produced by adding any of the above-described initiator tRNAs charged with an amino acid charged to the translation system. As described above, in the cell-free translation system, the initiator tRNA is recognized by the translation initiation factor IF2 in a case where a modified amino acid is charged thereto, and the translation is initiated. In addition, in a case where the peptide is charged to the initiator tRNA, the initiator tRNA can be recognized by the translation initiation factor IF2 even in a case where the peptide is unmodified.

Therefore, in the production method of the present embodiment, it is preferable that the translation system comprises any of the above-described initiator tRNAs, to which a modified amino acid is charged and/or a peptide is charged. Examples and preferred aspects of the modification of the amino acid charged to the initiator tRNA are as described above. The amino acid constituting the peptide charged to the initiator tRNA may or may not be modified.

In the present specification, the term "amino acid" is used in the broadest sense, and comprises not only a natural amino acid but also an artificial amino acid mutant or derivative. In addition, in the present specification, the term "amino acid" comprises proteinogenic amino acids and non-proteinogenic amino acids.

The proteinogenic amino acids mean 19 natural proteinogenic L-amino acids and glycine, and are represented by a three-letter notation generally known in the related art, the proteinogenic amino acids are Arg, His, Lys, Asp, Glu, Ser, Thr, Asn, Gln, Cys, Gly, Pro, Ala, Ile, Leu, Met, Phe, Trp, Tyr, and Val. In addition, in a case where the proteinogenic amino acids are represented by one-letter notation generally known in the related art, the proteinogenic amino acids are R, H, K, D, E, S, T, N, Q, C, G, P, A, I, L, M, F, W, Y, and V.

Non-proteinogenic amino acids mean natural or unnatural amino acids other than proteinogenic amino acids.

Examples of the non-proteinogenic amino acids comprise non-natural amino acids; chemically synthesized compounds having properties known in the related art as properties of an amino acid; and the like.

Examples of the non-proteinogenic amino acids comprise modified proteinogenic amino acids; β-amino acids, γ-amino acids, D-amino acids, and modified amino acids thereof; amino acids in which the structure of a main chain or a side chain is different from that of a natural type and modified amino acids thereof; and the like.

The aspect in a case where the amino acid is modified is not particularly limited, and examples thereof comprise modification of a heteroatom, and modification of a nitrogen atom, preferably a nitrogen atom of an amino group or an imino group, and more preferably a nitrogen atom at the α-position, the β-position, or the γ-position. More specifically, it is preferable that a nitrogen atom of an amino group is modified in a case of a primary amino acid and a nitrogen atom of an imino group is modified in a case of a secondary amino acid such as proline, and it is more preferable that a nitrogen atom of an amino group or an imino group at an α-position is modified in an α-amino acid, a nitrogen atom of an amino group or an imino group at a β-position is modified in a β-amino acid, and a nitrogen atom of an amino group or an imino group at a γ-position is modified in a γ-amino acid. Examples of the group to be bonded by modification comprise an alkyl group which may have a substituent and an acyl group which may have a substituent, and an alkyl group which may have a substituent, an acetyl group which may have a substituent, and a formyl group are preferable. The substituent is not particularly limited, and examples thereof comprise an alkyl group (preferably a C1 to C3 alkyl group) and a halogen atom (preferably a chlorine atom).

Examples of the amino acids in which the structure of a main chain is different from that of the natural type comprise α,α-disubstituted amino acids such as α-methylalanine and α-hydroxy acids.

Examples of the amino acids in which the structure of a side chain is different from that of the natural type comprise norleucine, homohistidine, and the like. The non-proteinogenic amino acid is not particularly limited, but may further comprise amino acids having an extra methylene in a side chain such as a "homo" amino acid, for example, homophenylalanine and homohistidine, or amino acids in which a carboxylic acid functional group in a side chain is substituted with a sulfonic acid group, such as cysteic acid. Specific examples of the unnatural amino acids comprise amino acids described in WO2015/030014A.

The β-amino acids and the γ-amino acids also comprise cyclic β-amino acids and γ-amino acids, and also comprise amino acids in which an amino group and a carboxy group are bonded to the 1-position and the 2-position of an aromatic ring, respectively, and amino acids in which an amino group and a carboxy group are bonded to the 1-position and the 3-position of an aromatic ring, respectively.

In a case where any of the above-described initiator tRNAs is charged with the modified amino acid, the amino acid is not particularly limited, but is preferably L-proline, a D-amino acid (comprising D-proline), a β-amino acid, or a γ-amino acid.

In these amino acids, it is preferable that in each of the α-amino acid, the β-amino acid, and the γ-amino acid, a nitrogen atom at an α-position, a β-position, and a γ-position is modified. Examples of the D-amino acid comprise D-forms of 19 types of natural proteinogenic amino acid excluding glycine, and D-Trp and D-Tyr are preferable. Examples of the β-amino acid comprise 3-aminopropanoic acid, L- and D-β-homophenylglycine, and L-β-homoamino acids comprising one extra carbon atom in the main chain of 19 natural proteinogenic amino acids excluding glycine, and D-forms thereof, and L-β-homophenylglycine is preferable. Examples of the γ-amino acid comprise an amino acid having an amino group and a carboxy group at the meta position of a benzene ring, γ-aminobutyric acid, L-γ-amino acid comprising two extra carbon atoms in the main chain of 19 natural proteinogenic amino acids excluding glycine, and a D-form thereof, and 3-aminobenzoic acid is preferable.

The initiator tRNA is preferably charged with L-proline, D-amino acid, β-amino acid, or γ-amino acid in which a nitrogen atom at the α-position, the β-position, or the γ-position is modified, and more preferably charged with L-proline, D-amino acid, β-amino acid, or γ-amino acid in which a nitrogen atom at the α-position, the β-position, or the γ-position is acylated.

In the present embodiment, by the combination of the tRNA^{iniP} precharged with a non-proteinogenic amino acid such as L-proline, D-amino acid, β-amino acid, or γ-amino acid, in which a nitrogen atom at an α-position, a β-position, or a γ-position is modified, and EF-P, it is possible to enhance the incorporation of various amino acids into the N terminus, the various amino acids comprising L-proline, D-amino acid, β-amino acid, γ-amino acid, or the like in which a nitrogen atom at an α-position, a β-position, or a γ-position is modified.

In a case where any of the above-described initiator tRNAs is charged with the peptide, the peptide is not particularly limited, and examples thereof comprise a peptide in which a plurality of amino acids in the above-described definition are bonded by a peptide bond. The peptide is preferably composed of 2 or more or 3 or more amino acids, and more preferably composed of 3 or more and 20 or less, 3 or more and 15 or less, 3 or more and 10 or less, 3 or more and 5 or less, 5 or more and 20 or less, 5 or more and 15 or less, 5 or more and 10 or less, 10 or more and 20 or less, or 10 or more and 15 or less amino acids. The peptide is preferably a linear peptide.

In the present embodiment, the 3'-end of the initiator tRNA has a 5'-CCA-3' sequence and may be bonded to an amino acid or a peptide. Examples of the amino acid or peptide comprise the above-described amino acid or peptide.

In the production method of the present embodiment, the method of charging the amino acid and the peptide to the above-described initiator tRNA is not particularly limited, and a known method in the related art may be used. For example, the charge of the amino acid and the peptide may be performed by a method using an artificial aminoacylating ribozyme Flexizyme.

In the production method of the present embodiment, the modified amino acid may be charged to the initiator tRNA, or the unmodified amino acid may be charged to the initiator tRNA and then the charged amino acid may be modified. The charge of the amino acid and/or the modification of the amino acid may be performed after the initiator tRNA is added to the translation system or may be performed before the addition. From the viewpoint of increasing the charge efficiency of the amino acid, it is preferable to modify the amino acid after charging the amino acid to the initiator tRNA. From the viewpoint of improving the ease of operation, it is preferable to directly charge the modified amino acid to the initiator tRNA.

The production method of the present embodiment comprises translating in a cell-free translation system using any of the above-described initiator tRNAs. The cell-free translation system of the present embodiment (hereinafter, also simply referred to as a "translation system") comprises the initiator tRNA, whereby various amino acids comprising proline and a non-proteinogenic amino acid can be efficiently introduced at the N-terminus of a peptide.

In the present specification, the term "cell-free translation system" refers to a translation system that does not comprise cells, and as the cell-free translation system, for example, an Escherichia coli extract, a wheat germ extract, a rabbit erythrocyte extract, an insect cell extract, or the like can be used. In addition, a reconstituted cell-free translation system may be used, which is constructed by reconstituting each of the purified ribosome, the aminoacyl tRNA synthetase (aaRS), the ribosomal RNA, the amino acid, the rRNA, the GTP, the ATP, the translation initiation factor (IF), the elongation factor (EF), the release factor (RF), the ribosome recycling factor (RRF), and other factors necessary for translation

To perform transcription from DNA at the same time, the cell-free translation system may be a system comprising RNA polymerase. As a commercially available cell-free translation system, for a system derived from Escherichia coli, RTS-100 (registered trademark) manufactured by Roche Diagnostics, for a reconstituted translation system, PURESYSTEM (registered trademark) manufactured by PGI, PURExpressR in Vitro Protein Synthesis Kit manufactured by New England Biolabs Inc., and the like, and for a system using a wheat germ extract, systems manufactured by Zoigen Co., Ltd. and CellFree Sciences, Co., Ltd. can be used.

In addition, as a system using a ribosome of Escherichia coli, for example, the following techniques are known: H. F. Kung et al., 1977, The Journal of Biological Chemistry Vol. 252, No. 19, 6889-6894; M. C. Gonza et al., 1985, Proceeding of National Academy of Sciences of the United States of America Vol. 82, 1648-1652; M. Y. Pavlov and M. Ehrenberg, 1996, Archives of Biochemistry and Biophysics Vol. 328, No. 1, 9-16; Y. Shimizu et al., 2001, Nature Biotechnology Vol. 19, No. 8, 751-755; H. Ohashi et al., 2007, Biochemical and Biophysical Research Communications Vol. 352, No. 1, 270-276.

According to the cell-free translation system, an expression product can be obtained in a highly pure form without being purified. It is noted that the cell-free translation system of the present embodiment may be used not only for translation but also for transcription by adding a factor required for transcription.

As the production method of a peptide using a cell-free translation system, for example, Flexible In vitro Translation system (FIT system) of the method described in Goto, Y., Katoh, T. & Suga, H. Flexizymes for genetic code reprogramming. Nat Protoc 6, 779-790, (2011) may be used.

The translation system used in the production method of the present embodiment is not particularly limited as long as it comprises the initiator tRNA of the present embodiment, and may comprise any component used in the cell-free translation system.

It is preferable that the translation system comprise an EF-P protein. In addition, it is preferable that the translation system comprise other components used in the well-known cell-free translation system in the related art.

In the translation system of the present embodiment, in addition to the initiator tRNA of the present embodiment, an elongator tRNA that encodes a proteinogenic amino acid or a non-proteinogenic amino acid may be further comprised. As long as the base at the first position of the elongator tRNA forms a base pair, other nucleotide sequence thereof is not particularly limited. It is preferable that in the elongator tRNA, the base at the first position is complementary to a base corresponding to the base at the 72nd position of the wild-type elongator tRNA in a case of being aligned with the wild-type elongator tRNA.

The concentration of each component in the translation system may be appropriately adjusted depending on the type of the peptide to be produced, the type of the initiator tRNA, and the like. For example, the following concentrations may be referred to for each component.
IF3: 4 to 50 µM (comprising both end values)
EF-G: 0.5 to 12 µM (comprising both end values)
RRF: 0.5 to 10 µM (comprising both end values)
EF-P: 2 to 50 µM (comprising both end values)
Aminoacyl-initiator tRNA: 20 to 200 µM (comprising both end values)

In the translation system in the present embodiment, it is preferable to assign an amino acid or a peptide to a codon appropriately selected as NNN in the reassignment of the codon using Flexizyme.

In the translation system in the present embodiment, a natural translation system may be used. In the natural translation system, there is a tRNA having an anticodon corresponding to each amino acid, and each tRNA has a unique sequence even in a region other than the anticodon loop.

In the present embodiment, first, NNN to which an amino acid or peptide to be introduced at the N-terminus is assigned is selected. Next, the initiator tRNA of the present embodiment, which has an NNN anticodon in the anticodon loop and is charged with an amino acid or peptide to be introduced at the N-terminus, is prepared using Flexizyme or the like. The initiator tRNA charged with the non-proteinogenic amino acid may be obtained by modifying the amino acid charged to the initiator tRNA as necessary.

Next, by preparing an mRNA comprising NNN, which encodes an amino acid or peptide to be introduced at the N-terminus, as an initiation codon and translating, a peptide comprising the amino acid or peptide assigned to the initiation codon at the N-terminus can be expressed.

In the present specification, "NNN" means a codon that designates an amino acid, and three Ns forming the codon are each independently selected from adenine (A), guanine (G), cytosine (C), and uracil (U). The mRNA may comprise NNN, which encodes an amino acid or peptide to be introduced at the N terminus, as an initiation codon.

In the present embodiment, any amino acid may be re-assigned to NNN that does not encode the amino acid or peptide to be introduced at the N terminus, or a relationship between a codon and an amino acid based on a natural genetic code may be used.

In reassignment, it is possible to assign a relationship between a codon and an amino acid that is different from that in the natural genetic code table, or to assign the same relationship.

The "natural genetic code table" is a table showing amino acids assigned to triplets (codons) of mRNA in a living body, and is a table shown in Table 1 below.

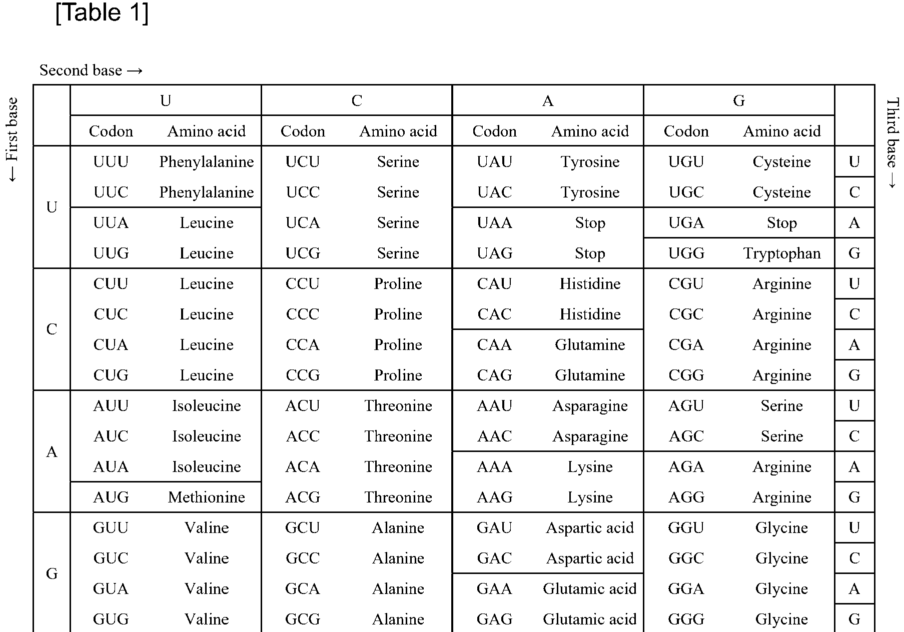

The assignment of amino acids different from the natural genetic code table for each codon is realized by, for example, the above-described codon reassignment using Flexizyme. According to Flexizyme, since a desired amino acid can be bonded to a tRNA having any anticodon, any amino acid can be assigned to any codon.

According to the production method of the present embodiment, it is possible to produce a peptide while suppressing N-terminal drop-off-reinitiation. The N-terminal of the peptide to be produced is preferably an amino acid in which a nitrogen atom is modified, more preferably L-proline, a D-amino acid, a β-amino acid, or a γ-amino acid in which a nitrogen atom at an α-position, a β-position, or a γ-position is modified, and particularly preferably L-proline, a D-amino acid, a β-amino acid, or a γ-amino acid in which a nitrogen atom at an α-position, a β-position, or a γ-position is acylated. In these amino acids, it is preferable that in each of the α-amino acid, the β-amino acid, and the γ-amino acid, a nitrogen atom at an α-position, a β-position, and a γ-position is modified.

The number of amino acids constituting the peptide to be produced is not particularly limited, and may be, for example, 2 or more and 100 or less. The number of amino acids may be 3 or more, 4 or more, 5 or more, 6 or more, 7 or more, 8 or more, 9 or more, 10 or more, 15 or more, or 20 or more, in the above-described range. The number of amino acids may be 90 or less, 80 or less, 70 or less, 60 or less, 50 or less, 40 or less, 35 or less, 30 or less, 25 or less, 20 or less, 19 or less, 18 or less, 17 or less, 16 or less, 15 or less, 14 or less, 13 or less, 12 or less, 11 or less, or 10 or less, in the above-described range. The peptide to be produced may be linear or cyclic. The peptide to be produced may comprise a portion other than the peptide chain consisting of amino acids, and such a portion is not particularly limited, but examples thereof comprise a group used for detecting or separating a peptide, and a nucleic acid such as mRNA and DNA. The peptide to be produced may comprise mRNA and/or DNA encoding the peptide.

In the present embodiment, the above-described initiator tRNA is also provided. The initiator tRNA of the present embodiment may have the same configuration as the initiator tRNA used in the above-described production method of a peptide, and the above-described aspects may be optionally combined. As long as the initiator tRNA of the present embodiment comprises the nucleotide sequence set forth in SEQ ID NO: 1, other nucleotide sequences of the initiator tRNA are not limited, but from the viewpoint that various amino acids comprising proline and a non-proteinogenic amino acid can be more efficiently introduced at the N-terminus of the peptide, it is preferable that the initiator tRNA comprises one or more nucleotide sequences set forth in SEQ ID NOs: 2 to 31 and 243 to 248. Furthermore, the initiator tRNA of the present embodiment preferably has an acceptor stem, a D-arm, an anticodon stem, an anticodon loop, a variable loop, and a T-arm, and the above-described aspects may be optionally combined for each of the acceptor stem, the D-arm, the anticodon stem, the anticodon loop, the variable loop, and the T-arm. The acceptor stem, the D-arm, the anticodon stem, the anticodon loop, the variable loop, and the T-arm may be the preferred nucleotide sequence, the more preferred nucleotide sequence, or the particularly preferred nucleotide sequence, as described for each. In addition, for example, a preferred nucleotide sequence may be selected as the acceptor stem, and a more preferred nucleotide sequence may be selected as the D-arm.

From the viewpoint of promoting peptidyl transfer by EF-P, the suitable initiator tRNA of the present embodiment preferably comprises, in addition to the nucleotide sequence set forth in SEQ ID NO: 1, any of the nucleotide sequence set forth in SEQ ID NO: 2, one nucleotide sequence selected from SEQ ID NOs: 3 to 6, the nucleotide sequence set forth in SEQ ID NO: 7, or the nucleotide sequence set forth in SEQ ID NO: 243, and more preferably comprises, in addition to the nucleotide sequence set forth in SEQ ID NO: 1, all of the nucleotide sequence set forth in SEQ ID NO: 2, one nucleotide sequence selected from SEQ ID NOs: 3 to 6, the nucleotide sequence set forth in SEQ ID NO: 7, and the nucleotide sequence set forth in SEQ ID NO: 243. The preferred aspects of the nucleotide sequence set forth in SEQ ID NO: 2, the nucleotide sequences set forth in SEQ ID NOs: 3 to 6, the nucleotide sequence set forth in SEQ ID NO: 7, and the nucleotide sequence set forth in SEQ ID NO: 243 are as described above, and preferred nucleotide sequences, more preferred nucleotide sequences, particularly preferred nucleotide sequences, as described for each, can be optionally combined.

The initiator tRNA of the present embodiment may or may not be charged with an amino acid or a peptide. In addition, in a case where the amino acid or the peptide is charged thereto, the amino acid may or may not be modified. Examples of the charged amino acid and peptide, and the modified aspect thereof comprise the above-described aspects.

In the present embodiment, a translation system comprising the initiator tRNA of the present embodiment is also provided. In the present embodiment, in a case of using the translation system of the present embodiment, an amino acid or a peptide is charged to the initiator tRNA of the present embodiment to prepare the initiator tRNA charged with the amino acid or the peptide. In a case where the initiator tRNA charged with the unmodified amino acid is prepared, the amino acid may be modified in the translation system.

By translating the mRNA library using the initiator tRNA and/or the translation system of the present embodiment, it is possible to produce a peptide library and a complex library of a peptide and an mRNA encoding the peptide. That is, in the present embodiment, a method for producing a peptide library, comprising a step of translating using a translation system comprising the initiator tRNA of the present embodiment, and a complex library of a peptide and an mRNA encoding the peptide, comprising a step of translating using a translation system comprising the initiator tRNA of the present embodiment. In the method of producing a peptide library and the method of producing a complex library of a peptide and an mRNA encoding the peptide, a translation system comprising the initiator tRNA of the present embodiment (in particular, a initiator tRNA charged with an amino acid or a peptide) is prepared. In the translation system, it is preferable to comprise EF-P.

In the method for producing a peptide library and the method for producing a complex library of a peptide and an mRNA encoding the peptide of the present embodiment, it is preferable to comprise a step of translating in a cell-free translation system using the initiator tRNA or the translation system of the present embodiment, and to comprise a step of preparing the translation system of the present embodiment before the translation in the cell-free translation system.

The proline and the non-proteinogenic amino acid are efficiently incorporated into the N-terminus of the peptide by charging the proline and the non-proteinogenic amino acid to the initiator tRNA of the present embodiment and translating by the cell-free translation system.

To improve the efficiency of translation, it is preferable that the affinity of the initiator tRNA charged with proline or a non-proteinogenic amino acid to EF-P is higher than the affinity of a tRNA other than the initiator tRNA to EF-P.

In the method for producing a peptide library of the present embodiment, it is preferable to comprise a step of preparing an mRNA library comprising NNN encoding an amino acid or a peptide and a step of translating each mRNA of the mRNA library in a cell-free translation system to which the initiator tRNA of the present embodiment having an anticodon for a codon of NNN and charged with an amino acid or peptide corresponding to the codon is added. The method may comprise a step of modifying the amino acid charged to the initiator tRNA, as necessary.

The method for producing a peptide-mRNA complex library of the present embodiment is performed by binding puromycin to a downstream region of an ORF (an open reading frame) of each mRNA in a case of preparing the mRNA library in the above-described method for producing a peptide library.

The puromycin may be bonded to the mRNA via a linker composed of a peptide and/or a nucleic acid. By binding puromycin to the downstream region of the ORF of mRNA, a ribosome that translates the ORF of mRNA incorporates the puromycin, and a complex of mRNA and peptide is formed. Such a peptide-mRNA complex can associate a genotype with a phenotype and can be applied to an in vitro display.

In the present embodiment, a peptide library or a peptide-mRNA complex library is also provided.

By producing a peptide library using the initiator tRNA of the present embodiment, it is possible to synthesize a peptide having the same expression level as that of a peptide consisting of L-amino acids and comprising proline or a non-proteinogenic amino acid at the N-terminus. Therefore, it is possible to obtain a peptide library having a rich diversity in the introduction of non-proteinogenic amino acids, as compared with the peptide library in the related art.

In addition, by producing a peptide-mRNA complex library using the initiator tRNA of the present embodiment, it is possible to synthesize a peptide-mRNA complex having the same expression level as that of a peptide consisting of an L-amino acid and comprising proline or a non-proteinogenic amino acid at N-terminus of the peptide. Therefore, it is possible to obtain a peptide-mRNA complex library having a rich diversity in the introduction of non-proteinogenic amino acids, as compared with the peptide-mRNA complex library in the related art.

In the present embodiment, the peptide library or the peptide-mRNA complex library preferably comprises a peptide comprising, at an N-terminus, L-proline, D-amino acid, β-amino acid, or γ-amino acid, in which a nitrogen atom at an α-position, a β-position, or a γ-position is modified, and more preferably comprises a peptide comprising, at an N-terminus, L-proline, D-amino acid, β-amino acid, or γ-amino acid, in which a nitrogen atom at an α-position, a β-position, or a γ-position is acylated.

As shown in Examples described later, the present invention shows that EF-P promotes the incorporation of various N-terminal substrates. The present inventors have previously reported that EF-P recognizes a specific D-arm motif of a tRNA^{Pro} isoacceptor. The D-arms of the tRNA^{fMet2} and the tRNA^{Pro1} are almost the same except for the base pair between the bases at positions 11 and 24 in the D-stem (the base pair of A/U in the tRNA^{fMet2} and the base pair of C/G in the tRNA^{Pro1}). Therefore, it is reasonable that the substitution of the A/U base pair of tRNA^{iniC1G} with the C/G base pair results in a significant improvement in the level and proportion of P1-FLP due to EF-P. The base pair of pyrimidine 11/purine 24 is unique and is widely preserved in prokaryotic initiator tRNA. However, it has been reported that a mutant initiator tRNA having a C/G base pair at this position exhibits extremely high activity in protein synthesis in vivo. Similarly, the C/G mutation in the artificial tRNA according to the embodiment of the present invention also allowed the incorporation of AcPro. In the present invention, the efficient incorporation of AcPro at the N-terminus was successful at an expression level of 1,000 times as compared with the wild type tRNA^{iniWT} by using tRNA^{iniP}. Furthermore, the N-terminal drop-off-reinitiation can be completely suppressed.

The N-terminal Pro residue is an attractive component of the bioactive foldamer peptide due to the constraint of the cyclic structure. It has been reported that the N-terminal Pro contributes to the stabilization of the turn conformation and the helical conformation of the peptide. In addition, β-amino acids, γ-amino acids, and D-amino acids are also useful components of the bioactive peptide that can be introduced by the present invention. Since these amino acids often exhibit unique and strong folding properties such as turn/helix induction ability, a peptide comprising these amino acids can be folded into various structures having properties closer to those of a drug. Furthermore, macrocyclization is a powerful approach for constructing a constrained shape of a peptide. The present invention also shows that the artificial initiator tRNA can introduce an N-chloroacetyl substrate for cyclization at the N-terminus of the peptide. In these foldamer peptides, high binding affinity and inhibitory activity for a target molecule, improvement of membrane permeability, and protein degradation stability are expected. The peptide consisting of only L-α-amino acid is rapidly degraded by a peptidase in serum and in cells, whereas the introduction of a special amino acid at the N-terminus results in a dramatic improvement in protein degradation stability against exopeptidase. Furthermore, the protein degradation stability is also improved by N-acetylation.

The advantage of synthesizing such an exogenous peptide with a ribosome is that a random peptide library can be easily prepared by translating an mRNA having a random sequence, and the library can be applied to a display-based screening method such as a RaPID (a random nonstandard peptides integrated discovery) system. According to the present invention, it is possible to perform RaPID screening of a novel bioactive peptide having an exogenous amino acid at an N-terminus.

### Examples

Hereinafter, the present embodiment will be described in more detail with reference to Examples and Comparative Examples, but the present embodiment is not limited to these Examples. Those skilled in the art can change the present invention to various aspects without departing from the spirit of the present invention, and such changes are also comprised in the scope of the present invention.

### Preparation of tRNAⁱⁿⁱ mutant and Flexizyme

The tRNAⁱⁿⁱ mutant and the template DNA of the Flexizymes (dFx and eFx) were prepared by extension of a forward and reverse extension primer pair, and then PCR was performed using the forward and reverse extension primer pair (see Table 2 for sequences of dFx and eFx and used primers).

In a reaction mixture of 250 µL and 2 mL comprising 40 mM Tris-HCl (pH 8.0), 22.5 mM MgCl₂, 1 mM DTT, 1 mM spermidine, 0.01% Triton X-100, 3.75 mM or 5 mM nucleoside triphosphate (NTP) mix, 5 mM or 0 mM guanosine monophosphate (GMP), 0.04 U/µL Rnasin RNase inhibitor (Promega), and 0.12 µM T7 RNA polymerase, transcription of tRNAⁱⁿⁱ and the Flexizyme was performed overnight at 37°C. 200 µL or 2 mL scale PCR products were each added to the reaction mixture for the transcription of tRNAⁱⁿⁱ and Flexizymes. The concentration of NTPmix was 3.75 mM for tRNAⁱⁿⁱ and 5 mM for Flexizymes, and the concentration of GMP was 5 mM and 0 mM, respectively. The transferred tRNAⁱⁿⁱ and Flexizyme were treated with RQ1 DNase (Promega) at 37°C for 30 minutes, and then purified with an 8% (tRNAⁱⁿⁱ) or 12% (Flexizymes) polyacrylamide gel comprising 6M urea.

The obtained RNA was eluted from the gel, precipitated with ethanol, and dissolved in water.

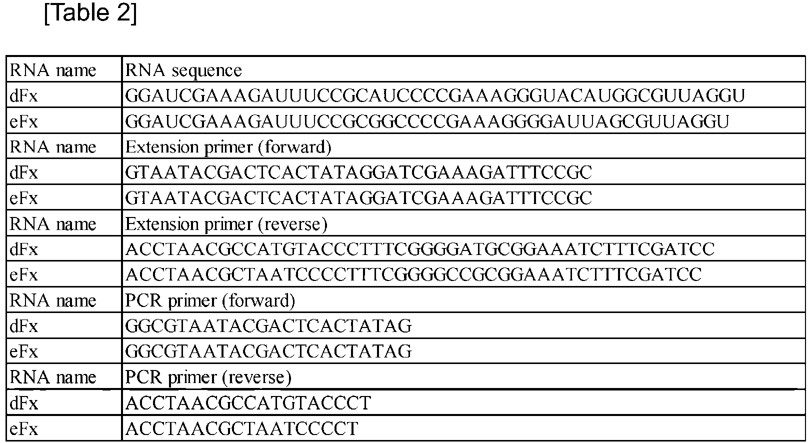

The nucleotide sequences shown in Table 2 correspond respectively to SEQ ID NOs: 32 to 41 in order from the top.

### Preparation of aminoacyl-tRNA

Aminoacylation of the tRNAⁱⁿⁱ mutant was performed at 0°C in a reaction mixture comprising 50 mM HEPES-KOH (pH 7.5), Bicine-KOH (pH 9.0) or CHES-KOH (pH 10.0), 600 mM MgCl₂, 20% dimethyl sulfoxide (DMSO), 25 µM dFx or eFx, 25 µM tRNAⁱⁿⁱ mutant, and 5 mM activated amino acid. To charge the tRNAⁱⁿⁱ mutants with Pro, Ac^{D}Tyr, Ac^{D}Trp, ^{β}Phg, and ³Abz, L-proline 3,5-dinitrobenzyl ester (Pro-DBE), N-acetyl-D-tyrosine-cyanomethyl ester (Ac^{D}Tyr-CME), N-acetyl-D-tryptophan cyanomethyl ester (Ac^{D}Trp-CME), L-β-homophenylglycine-3,5-dinitrobenzyl ester (^{β}Phg-DBE), and 3-aminobenzoic acid-cyanomethyl ester (³Abz-CME) were used as activated amino acids. The reactions were performed for 2, 3, 3, 22, and 144 hours, respectively. The acylation of ^{β}Phg and ³Abz was performed at pH 9.0 and pH 10.0, respectively, and other substrates were acylated at pH 7.5. These activated amino acids were synthesized according to the methods described in References 1 to 3 described later. dFx was used for DBE, and eFx was used for CME. The obtained aminoacyl-tRNA was precipitated with ethanol, washed twice with 0.1 M sodium acetate (pH 5.2), and then dissolved in 1 mM sodium acetate (pH 5.2). For the acetylation of Pro-tRNAⁱⁿⁱ, ^{β}Phg-tRNAⁱⁿⁱ, and ³Abz-tRNAⁱⁿⁱ, 250 pmol of aminoacyl-tRNAⁱⁿⁱ was dissolved in 60 µL of 0.3 M sodium acetate/0.5 M acetic anhydride solution (pH 5.2), incubated at 25°C for 30 minutes, and then recovered by ethanol precipitation. The obtained N-acetyl-aminoacyl-tRNA was washed with 70% ethanol comprising 0.1 M sodium acetate (pH 5.2) and then dissolved in 1 mM sodium acetate (pH 5.2). For N-chloroacetylation of the Pro-tRNAⁱⁿⁱ and ³Abz-tRNAⁱⁿⁱ, 500 pmol of aminoacyl-tRNAⁱⁿⁱ was dissolved in 75 µL of a 0.3 M sodium acetate/40 mM chloroacetic acid anhydride solution (pH 5.2), incubated at 25°C for 5 minutes, and then recovered by ethanol precipitation. The obtained N-acetyl- or N-chloroacetylaminoacyl-tRNA was washed twice with 70% ethanol (pH 5.2) comprising 0.1 M sodium acetate and dissolved in 1 mM sodium acetate (pH 5.2).

### Translation of model peptide

The model peptide P1 was translated using a template DNA encoding mRNAs mR1 to mR6. The template DNA was prepared by a forward and reverse extension primer pair, and then PCR was performed using the forward and reverse extension primer pair (see Table 3 for sequences of mRNAs mR1 to mR6, and Tables 4 and 5 for the used primers). Translation was performed at 37°C for 30 minutes in a 2.5 µL scale FIT (Flexible in vitro translation) system (Reference 4) using a composition comprising 50 mM HEPES-KOH (pH 7.6), 100 mM potassium acetate, 12.6 mM magnesium acetate, 2 mM ATP, 2 mM GTP, 1 mM CTP, 1 mM UTP, 20 mM creatine phosphate, 2 mM spermidine, 1 mM DTT, 1.5 mg/mL E. coli total tRNA, 1.2 µM E. coli ribosome, 0.6 µM methionyl-tRNA formyltransferase, 2.7 µM IF1, 3 µM IF2, 1.5 µM IF3, 0.1 µM EF-G, 20 µM EF-Tu/Ts, 0 µM or 5 µM EF-P, 0.25 µM RF2, 0.17 µM RF3, 0.5 µM RRF, 4 µg/mL creatine kinase, 3 µg/mL myokinase, 0.1 µM inorganic pyrophosphatase, 0.1 µM nucleotide diphosphate kinase, 0.1 µM T7 RNA polymerase, 0.13 µM AspRS, 0.11 µM LysRS, 0.02 µM TyrRS, 0.5 mM Asp, 0.5 mM U-¹³C:U-¹⁵N-Lys, 0.5 mM Tyr, 20 µM aminoacyl-tRNAⁱⁿⁱ mutant, 0.5 µM DNA template, and 0.5 µM internal control peptide (control-P1-FLP and control-P1-RiP). The sequences of Control-P1-FLP and Control-P1-RiP were the same as the sequences of Translation-P1-FLP and Translation-P1-RiP, respectively, except for the U-¹³C:U-¹⁵N-Lys label. In the titration of IF3, EF-G, EF-P, RRF, and aminoacyl-tRNAⁱⁿⁱ, the concentration was changed as shown in Figure 4. It is noted that in the experiment shown in Figure 6, the internal control peptide was not added, and U-¹³C:U-¹⁵N-Lys was used for translation in the presence of EF-P and was not used for translation in the absence of EF-P.

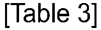

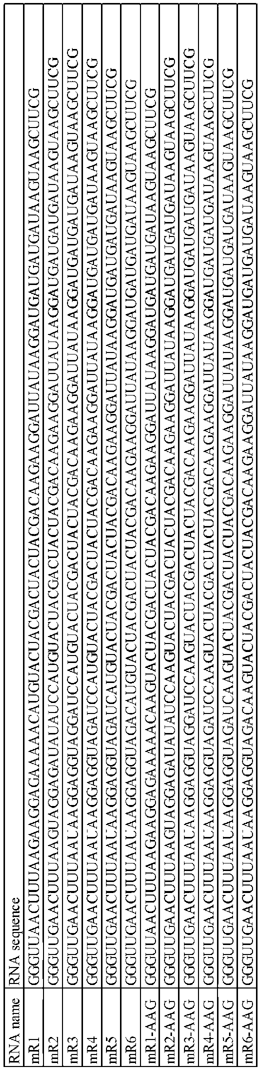

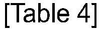

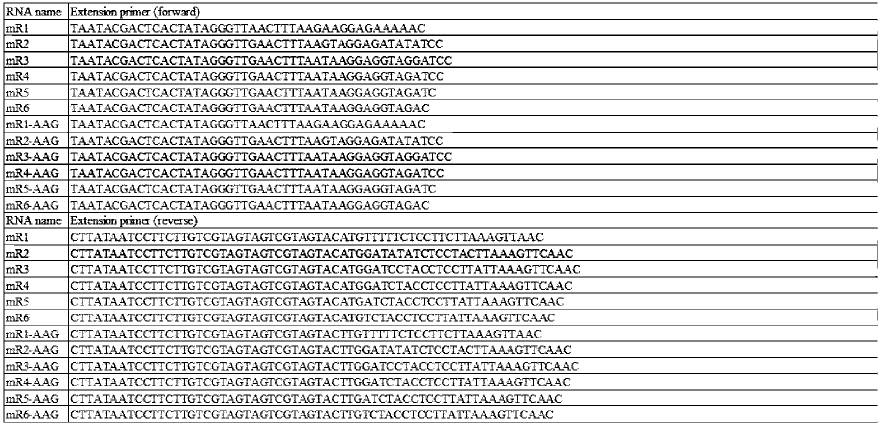

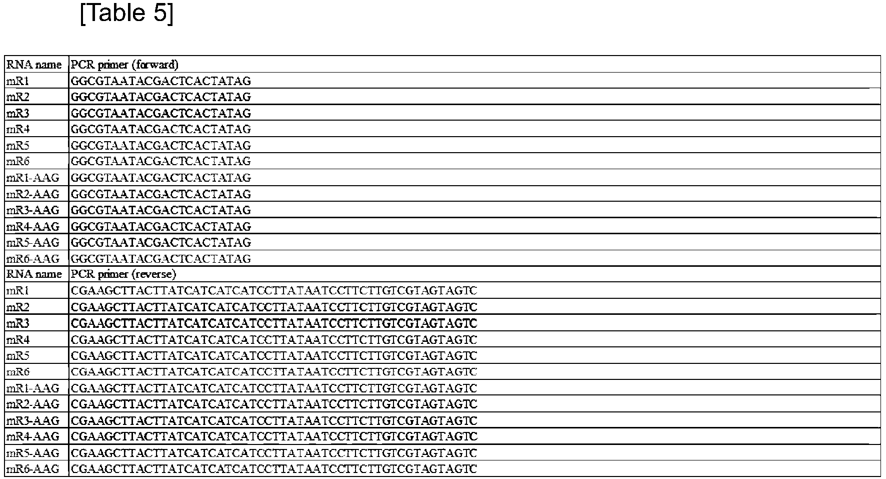

The nucleotide sequences shown in Table 3 each correspond to SEQ ID NOs: 177 to 188 in order from the top. In addition, the nucleotide sequences shown in Table 4 each correspond to SEQ ID NOs: 189 to 212 in order from the top. Furthermore, the nucleotide sequences shown in Table 5 each correspond to SEQ ID NOs: 213 to 236 in order from the top.

### MALDI-TOF mass spectrometry of model peptide

The translated peptide was desalted with SPE C-tip (Nikkyo Technos Co., Ltd.) and then co-crystallized with α-cyano-4-hydroxycinnamic acid on a sample plate. In the analysis shown in Figure 6, two translation solutions derived from the EF-P(+) and EF-P(-) experiments were mixed together, and then subjected to C-tip and co-crystallization. MALDI-TOF-mass spectrometry (MS) was performed using ultra fleXtreme (Bruker Daltonics) in a reflector-positive mode. A peptide mass standard II (Bruker Daltonics) was used for external mass calibration.

### Suppression of N-terminal drop-off-reinitiation by EF-P

To observe the N-terminal drop-off-reinitiation, acetylproline (AcPro) was introduced into the model peptide P1 using the mRNA mR1 (Figure 2A). To incorporate AcPro, three initiator tRNA mutants, tRNA^{iniWT}, tRNA^{iniC1G}, and tRNA^{iniC1G/A11C/U24G} were first tested (see upper left of Figure 1C, Figure 2C, Table 6 for the sequences of each tRNA, and Tables 7 and 8 for the primers used). All of these tRNAs are derived from Escherichia coli tRNA^{fMet2}, but they lack nucleotide modification due to in vitro transcription. In addition, the tRNA^{iniC1G} and the tRNA^{iniC1G/A11C/U24G} each have a C1G mutation and a C1G/A11C/U24G mutation. The C1G mutation was for the purpose of improving the transcription efficiency, and the A11C/U24G mutation was for the purpose of efficient recognition by EF-P. EF-P recognizes the D-arm of tRNA^{Pro1}, and since the only difference between the D-arms of tRNA^{fMet2} and tRNA^{Pro1} is observed at this position (Figure 1C, A11/U24 of tRNA^{fMet2} and C11/G24 of tRNA^{Pro1}), it was assumed that the A11C/U24G mutation should enhance the recognition by EF-P. Pro was precharged to these tRNAs using dFx, which is one of the Flexizyme mutants, and N-acetylated with acetic anhydride to prepare AcPro-tRNA.

The translation was performed in an E. coli reconstituted translation system called a FIT system, which comprises U-¹³C:U-¹⁵N-Lys instead of the non-labeled Lys. Therefore, the translated peptide is labeled with four U-¹³C:U-¹⁵N-Lys (Figure 2A, light gray portion). As a result, both the full-length P1 (P1-FLP) and the reinitiated peptide (P1-RiP) lacking the N-terminal AcPro were detected by MALDI-TOF-MS (Figure 2B, representative results of tRNA^{iniC1G/A11C/U24G} in the presence of EF-P). The expression levels of the translated P1-FLP and P1-RiP were estimated by their relative peak intensities with respect to the relative peak intensities of the 0.5 µM synthetic internal control peptides (control-P1-FLP and control-P1-RiP) comprising a non-labeled Lys. It was estimated that the translated peptide and the control P1 peptide had the same amino acid sequence except for the isotope labeling, thereby having the same ionization efficiency. As a result, the levels of P1-FLP using tRNA^{iniWT}, tRNA^{iniC1G}, and tRNA^{iniC1G/A11C/U24G} were 0.067 µM, 0.061 µM, and 0.87 µM, respectively, in the presence of EF-P, and were 0.045 µM, 0.038 µM, and 0.23 µM, respectively, in the absence of EF-P (Figure 2D). The percentages of P1-FLP [P1-FLP%: P1-FLP/(P1-FLP + P1-RiP) x 100] were 17%, 17%, and 34%, respectively in the absence of EF-P, and were 24%, 23%, and 43%, respectively, in the presence of EF-P (Figure 2D). The addition of EF-P resulted in an improvement of 1.5 to 1.6 times in the expression levels of tRNA^{iniWT} and tRNA^{iniC1G}, but resulted in an improvement of 3.8 times in the expression level of tRNA^{iniC1G/A11C/U24G}. These results indicate that the D-arm of the tRNA^{iniWT} and the tRNA^{inic1G} derived from the tRNA^{fMet2} was recognized by EF-P, and the A11C/U24G mutation enhanced the recognition of EF-P. On the other hand, the C1G mutation did not affect the function of the initiator tRNA. Therefore, thereafter, the C1G mutation was introduced into all the initiator tRNAs. Interestingly, the tRNA^{inicC1G/A11C/U24G} exhibited a significantly higher expression level and P1-FLP% than the tRNA^{iniWT} and the tRNA^{inicC1G} even in the absence of EF-P, and the change in the steric conformation induced by the A11C/U24G mutation was also found to be preferable for the incorporation of AcPro.

### Optimization of tRNAⁱⁿⁱ structure for more efficient incorporation of EF-P

The above results motivated further optimization of the local structure of tRNA^{inicC1G/A11C/U24G} and improvement of the P1-FLP level and the P1-FLP%. Here, five mutations of the anticodon stem (An1 to An5), five mutations of the acceptor stem (Ac1 to Ac5), four mutations of the T-stem (T1 to T4), four mutations of the variable loop (V1 to V4), and combinations thereof were introduced. Here, the sequence of tRNA^{Pro1} is partially embedded in tRNA^{inicC1G/A11C/U24G} (Figures 3, 7, and 8; the base derived from tRNA^{inicC1G} is shown in gray, the base derived from tRNA^{Pro1} is shown in dark gray, and the common base is shown in black). The tRNA^{inicC1G/A11C/U24G} has An1, Ac1, T1, and V1, and hearafter, is referred to as tRNA^{An1Ac1T1V1} (Figure 2C). Other mutants are similarly named tRNA^{AnXAcXTXVX} according to their local structures, where X indicates the numbering of the local structures shown in Figure 3A.

First, the mutation of the anticodon stem was evaluated (Figure 3B, Figure 7A, tRNA^{An2-5Ac1T1V1}. Among these, the tRNA^{An4Ac1T1V1} exhibited the highest level and ratio of P1-FLP in the presence of EF-P (Figure 3B, 0.98 µM and 58%). Although EF-P did not dramatically improve the expression level of P1-FLP of this tRNA (1.1 times), the P1-FLP% was increased from 39% to 58% by EF-P. In contrast, the other mutants exhibited relatively low expression levels and P1-FLP% (Figure 3B, tRNA^{An2Ac1T1V1}, tRNA^{An3Ac1T1V1}, and tRNA^{An5Ac1T1V1}, each of which was 0.30 to 0.97 µM and 7% to 34%). Therefore, it was concluded that An4 had the best anticodon stem structure among An1-5.

Next, the mutation of the acceptor stem in which the anticodon stem was fixed to An4 was evaluated (Figure 3C, Figure 7B, tRNA^{An4Ac2-5T1V1}). As a result, the tRNA^{An4Ac1T1V1} exhibited the highest P1-FLP level and P1-FLP% (Figure 3C, 0 to 0.24 µM and 0 to 14% in the presence of EF-P). Therefore, it was concluded that Ac1 had the most excellent acceptor stem structure among Ac1-5. Third, using tRNA^{An4Ac1T2-4V2-4} in which the anticodon stem and the acceptor stem were fixed to An4 and Ac1, respectively, a combination of the mutation in the T-stem and the mutation in the variable loop was evaluated (Figure 3D and Figure 8). As a mutation of the T-stem, T3 exhibited a generally higher P1-FLP level and P1-FLP% than T1, T2, and T4 (Figure 3D). As a mutation of the variable loop, V3 exhibited a higher P1-FLP level and P1-FLP% than V1, V2, and V4 (Figure 3D). Therefore, as in the case of P1-FLP%, the highest value of P1-FLP% was obtained by the combination of T3 and V3 (Figure 3D, in the presence of EF-P, 1.12 µM and 55%, respectively, improved by 1.6 times by EF-P).

Since the tRNA^{An4Ac1T3V3} exhibited the highest P1-FLP level among all the tRNAs evaluated in the present experiment, this tRNA was used in the subsequent experiment. Hereafter, tRNA^{An4Ac1T3V3} is referred to as tRNA^{iniP} (Figures 1C and 8) (see Table 6 for the sequences of the respective tRNAs, and Tables 7 and 8 for the primers used).

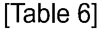

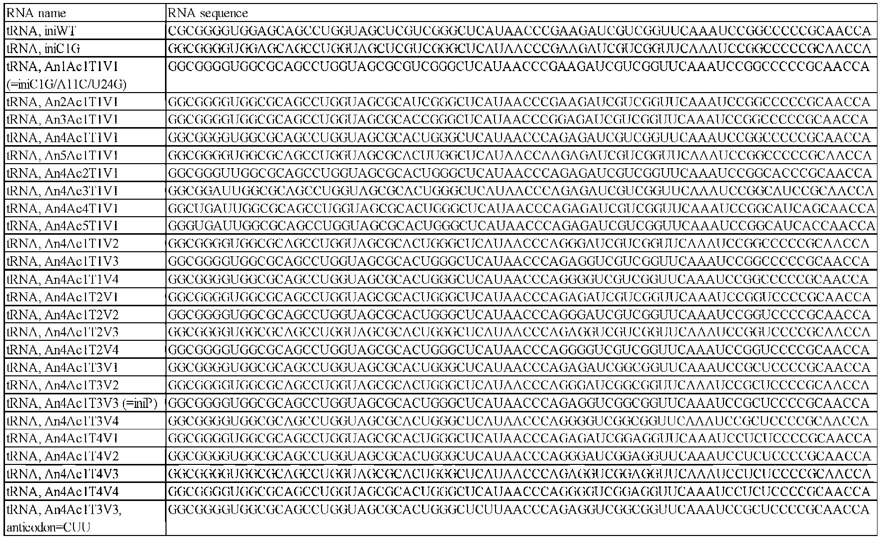

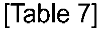

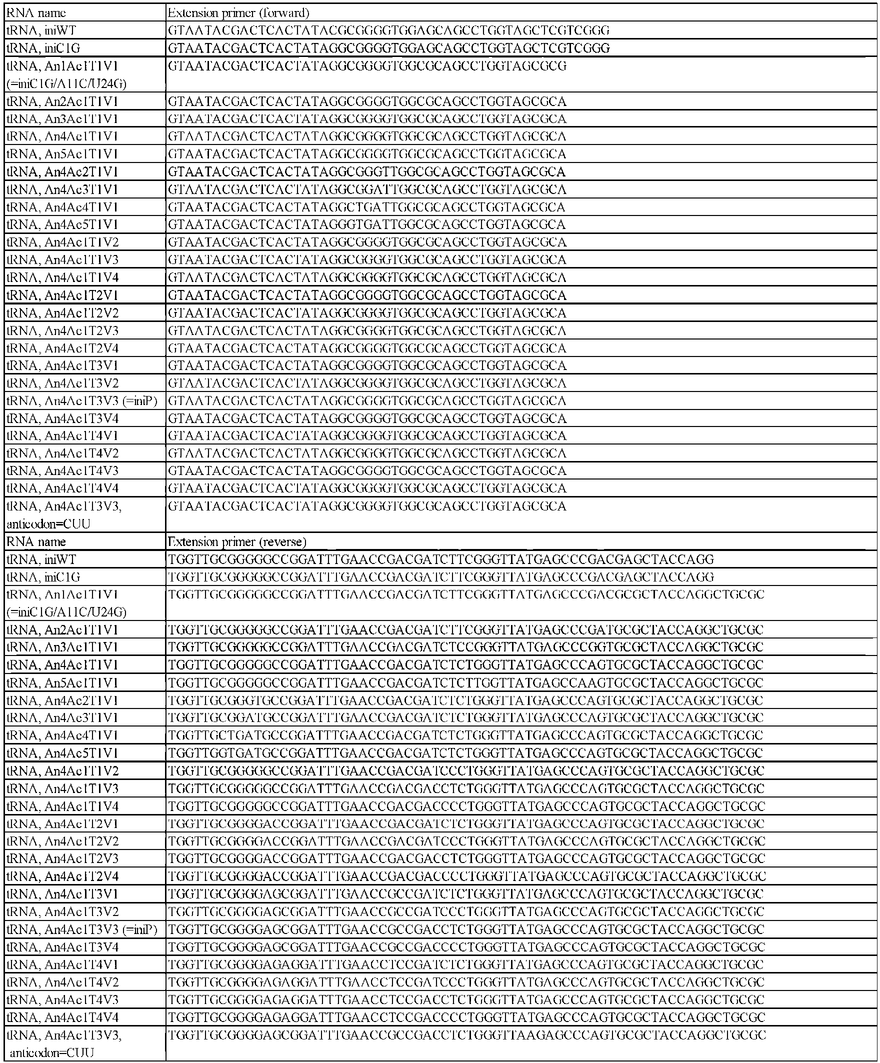

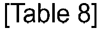

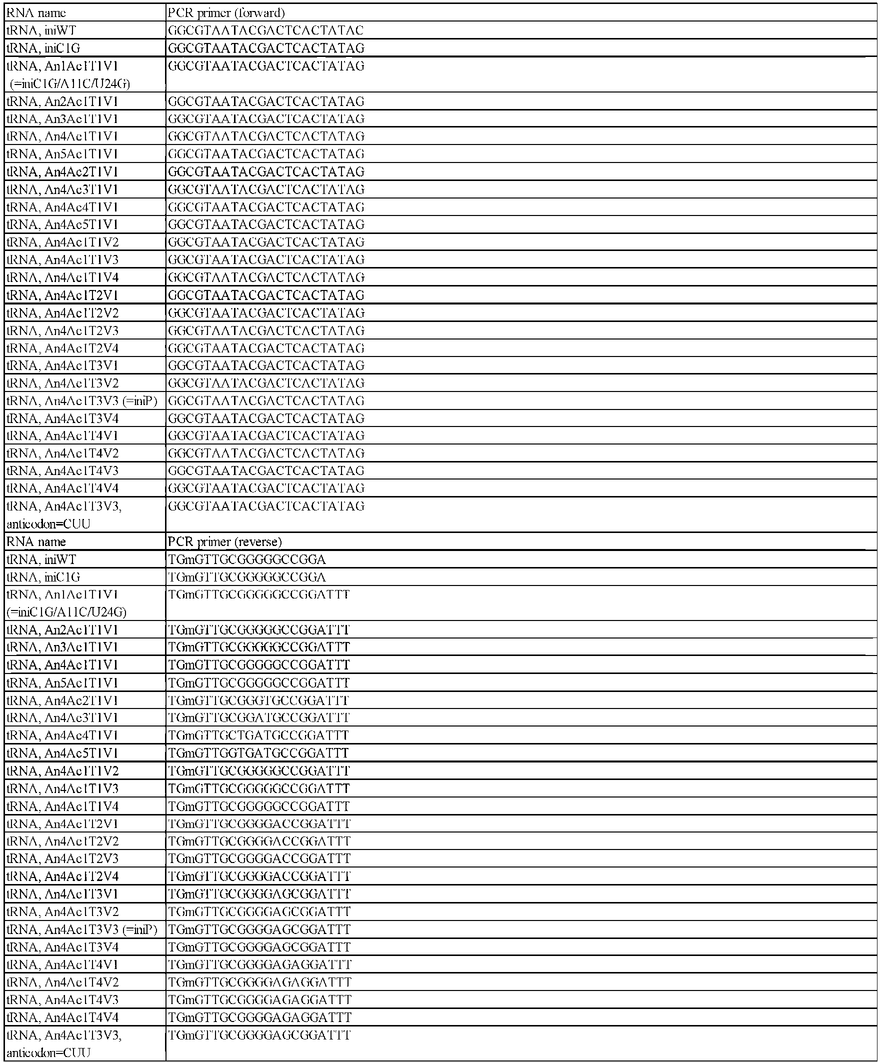

The nucleotide sequences shown in Table 6 each correspond to SEQ ID NOs: 42 to 68 in order from the top. In addition, the nucleotide sequences shown in Table 7 each correspond to SEQ ID NOs: 69 to 122 in order from the top. Furthermore, the nucleotide sequences shown in Table 8 each correspond to SEQ ID NOs: 123 to 176 in order from the top.

### Optimization of translation conditions for suppressing N-terminal drop-off-reinitiation

To enhance the P1-FLP level and the P1-FLP%, the concentrations of IF3, EF-G, and RRF were optimized (Figures 4A, 4B, and 4C). Furthermore, the concentrations of EF-P and AcPro-tRNA^{iniP} were also titrated for the P1-FLP synthesis (Figures 4D and 4E). In the titration of IF3, the P1-FLP concentration reached a plateau at IF3 having a concentration of 5 µM or more, and the P1-FLP% exceeded 90% at 15 µM or more (Figure 4A). Therefore, IF3 was used at 15 µM for the remaining experiments. In the titration of EF-G, the P1-FLP level reached a peak at 1 µM EF-G and P1-FLP% was 98%. The P1-FLP level gradually decreased (Figure 4B) in a case where the concentration of EF-G exceeded 1 µM. This indicates that in a case where the concentration of EF-G is too high, there is a possibility that erroneous transcription occurs frequently and AcPro-tRNA^{iniP} falls off from the P site. Regarding the concentrations of RRF and AcPro-tRNA^{iniP}, the P1-FLP level reached a plateau at 1 µM RRF and 80 µM AcPro-tRNA^{iniP} (Figure 4C and 4E). Regarding the concentration of EF-P, the P1-FLP level reached a peak at 5 to 10 µM EF-P and gradually decreased at a higher EF-P concentration (Figure 4D). This is considered to be because EF-P occupies the E site of the ribosome even after the peptidyl transfer has been completed, and the translocation of the deacylated tRNA from the P site to the E site is inhibited. In a case of comparing the P1-FLP level at 10 µM EF-P with the P1-FLP level at 0 µM EF-P, an improvement of 2.8 times was observed (Figures 4D, 21.1 µM and 7.6 µM, respectively), and it was shown that the tRNA^{iniP} was recognized by EF-P under these reaction conditions, and the peptidyl transfer was enhanced. Under the optimal translation conditions (Figure 4C, 15 µM IF3, 1 µM EF-G, 2.5 µM RRF, 10 µM EF-P, and 80 µM AcPro-tRNA^{iniP}), the P1-FLP level reached 25 µM, and at that point, the N-terminal drop-off-reinitiation was completely suppressed (P1-FLP% = 100).

For the incorporation of AcPro into P1, 9 combinations of codon-anticodon pairs was examined using tRNA^{iniP} (Figure 5A, codon:anticodon = AUU:AAU, AUU:GAU, AUC:GAU, AUA:UAU, AUG:CAU, AAG:CUU, AGA:UCU, GUA: UAC, and GGA:UCC). The anticodon of the tRNA^{iniP} was changed to recognize the corresponding codon sequence (see Table 6 for the sequence of each tRNA, and Tables 7 and 8 for the primers used). It is noted that in this analysis, the concentration of AcPro-tRNA^{iniP} was increased to 160 µM. As a result, AUU:GAU, AUC:GAU, AAG:CUU, and GUA:UAC exhibited a significantly higher P1-FLP level than the standard AUG:CAU (Figure 5A, 54.8 to 63.4 µM for AUU:GAU, AUC:GAU, AAG:CUU, and GUA:UAC; 25.1 µM for AUG:CAU), but AUU:GAU and AUC:GAU exhibited a decrease in P1-FLP% (89% and 91%, respectively; 99% to 100% for other combinations). Therefore, it was concluded that AAG:CUU and GUA:UAC are preferred combinations of codons and anticodons for P1-FLP synthesis.

Furthermore, the effects of the Shine-Dalgarno (SD) sequence and the spacer between the SD sequence and the initiation codon were evaluated. In addition to mR1, 5 SD+spacer sequences into which AUG and AAG were introduced as initiation codons were evaluated (Figure 5B, mR2 to mR6). As a result, a wide range of P1-FLP levels were observed depending on the type of SD+spacer. mR5 exhibited the highest P1-FLP for both AUG and AAG codons (Figure 5C, 0.39 to 59.1 µM (range of P1-FLP levels for all SD+spacer), 43.8 µM and 59.1 µM for mR5-AUG and mR5-AAG, respectively). Therefore, it was concluded that the use of mR5-AAG having 15 µM IF3, 1 µM EF-G, 2.5 µM RRF, 10 µM EF-P, and 160 µM AcPro-tRNA^{iniP} is the best condition for the translation of P1-FLP. Under these conditions, 59.1 µM P1-FLP was obtained, which was improved by 1,000 times or more as compared with a case where tRNA^{iniWT} was used (Figure 2C, tRNA^{iniWT}, EF-P(-), 0.045 µM).

### Ribosome incorporation of D-amino acid, β-amino acid, and y-amino acid at N-terminus using tRNA^{iniP} and EF-P

Using N-acetyl-D-tryptophan (Ac^{D}Trp), N-acetyl-D-tyrosine (Ac^{D}Tyr), N-acetyl-L-β-homophenylglycine (Ac^{β}Phg), and N-acetyl-3-aminobenzoic acid (Ac³Abz) as representative substances of D-amino acids, β-amino acids, and γ-amino acids, the purpose was the application of the tRNA^{iniP} to the incorporation of D-amino acids, β-amino acids, and γ-amino acids (Figure 6A). These amino acids were precharged to a tRNA^{iniP} having a CUU anticodon, which is represented by tRNA^{iniP}_{CUU}, and then introduced to the N terminus of the P1 peptide using mR5-AAG, thereby obtaining P1-FLP-Ac^{D}Trp, P1-FLP-Ac^{D}Tyr, P1-FLP-Ac^{P}Phg, and P1-FLP-Ac³Abz. A translation reaction was performed at 37°C for 30 minutes in 2.5 µL of a reaction solution comprising 15 µM IF3, 1 µM EF-G, 2.5 µM RRF, 160 µM aminoacyl-tRNA^{iniP}cuu, and 0 µM or 10 µM EF-P. U-¹³C:U-¹⁵N-Lys was added to the EF-P(+) reaction mixture, but non-labeled Lys was added to the EF-P(-) reaction. By adding EDTA and mixing, both reactions of EF-P(+) and EF-P(-) were stopped, and the mixture was subjected to MALDI-TOF-MS (Figure 6B). To evaluate the enhancement by EF-P, the peak intensities of U-¹³C:U-¹⁵N-Lys-labeled P1-FLP and non-labeled P1-FLP were compared. As a result, the expression levels of P1-FLP-AcPro, P1-FLP-Ac^{D}Trp, P1-FLP-Ac^{D}Tyr, P1-FLP-Ac^{β}Phg, and P1-FLP-Ac³Abz were improved by 4.1 times, 81 times, 390 times, 7.1 times, and 1.7 times, respectively. These results indicate that the tRNA^{iniP} can be applied to the enhancement of the incorporation of the D-amino acid, the β-amino acid, and the γ-amino acid into the N-terminus, similarly to the incorporation of AcPro.

N-chloroacetylated amino acid was used instead of N-acetylated amino acid for macrocyclization of the peptide. Here, N-chloroacetyl-L-Pro (ClAcPro) and N-chloroacetyl³Abz (ClAc³Abz) were precharged to tRNA^{iniP}_{CUU} and introduced into the N-terminus of the model peptide P7 (Figures 9A to 9C). Translation was performed in the presence of 15 µM IF3, 1 µM EF-G, 10 µM EF-P, 2.5 µM RRF, and 200 µM aminoacyl-tRNA^{iniP}_{CUU}. The N-chloroacetyl group spontaneously reacted with the sulfhydryl group of downstream Cys to form a thioether bond that was not reduced, and as a result, the macrocyclization of the peptide occurred. The MALDI-TOF MS of the translation product showed that the expected cyclic peptide was expressed without cleavage (Figures 9D to 9G, P7-FLP-ClAcPro and P7-FLP-ClAc³Abz).

Reference Literature 1: Saito, H., Kourouklis, D. and Suga, H. (2001) An in vitro evolved precursor tRNA with aminoacylation activity. EMBO J., 20, 1797-1806.
Reference Literature 2: Murakami, H., Ohta, A., Ashigai, H. and Suga, H. (2006) A highly flexible tRNA acylation method for non-natural polypeptide synthesis. Nat. Methods, 3, 357-359.
Reference Literature 3: Katoh, T. and Suga, H. (2020) Ribosomal elongation of aminobenzoic acid derivatives. J. Am. Chem. Soc., 142, 16518-16522.
Reference Literature 4: Katoh, T. et al. Cell Chem. Biol. 24, 46-54 (2017).

## Claims

1. A production method for a peptide comprising:
translating in a cell-free translation system using an initiator tRNA comprising a nucleotide sequence set forth in SEQ ID NO: 1,
GCGCN₁N₂N₃N₄N₅N₆N₇N₈N₉GCGC (SEQ ID NO: 1)
(in SEQ ID NO: 1, N₁ to N₉ each independently represent any base, N₁ to N₉ form a D-loop, and GCGC forms a base pair with GCGC).

2. The production method according to Claim 1,
wherein the peptide to be produced is a peptide comprising, at an N terminus, a D-amino acid, a β-amino acid, a γ-amino acid, or L-proline, each comprising a nitrogen atom modified at an α-position, a β-position, or a γ-position.

3. The production method according to Claim 1,
wherein the initiator tRNA is charged with a D-amino acid, a β-amino acid, a γ-amino acid, or L-proline, each comprising a nitrogen atom modified at an α-position, a β-position, or a γ-position, or with a peptide.

4. The production method according to Claim 1,
wherein the initiator tRNA further comprises a nucleotide sequence set forth in SEQ ID NO: 2,
N₁₀N₁₁N₁₂N₁₃GGN₁₄N₁₅N₁₆N₁₇N₁₈N₁₉N₂₀CCN₂₁N₂₂N₂₃ (SEQ ID NO: 2)
(in SEQ ID NO: 2, N₁₄ to N₂₀ each independently represent any base, N₁₀ represents A or G, N₁₁ represents C or U, N₁₂ represents C or U, N₁₃ represents G or U, N₂₁ represents A or C, N₂₂ represents A or G, N₂₃ represents A or G, N₁₄ to N₂₀ form an anticodon loop, and N₁₁N₁₂N₁₃GG forms a base pair with CCN₂₁N₂₂N₂₃).

5. The production method according to Claim 1,
wherein the initiator tRNA further comprises one nucleotide sequence selected from SEQ ID NOs: 3 to 6,
GUCGG(N₂₄)ₘCCGGC (SEQ ID NO: 3)
(in SEQ ID NO: 3, N₂₄'s each independently represent any base, m is an integer of 1 or more, (N₂₄)ₘ forms a T-loop, and GUCGG forms a T-stem with CCGGC)
GUCGG(N₂₅)ₘCCGGU (SEQ ID NO: 4)
(in SEQ ID NO: 4, N₂₅'s each independently represent any base, m is an integer of 1 or more, (N₂₅)ₘ forms a T-loop, and GUCGG forms a T-stem with CCGGU)
GGCGG(N₂₆)ₘCCGCU (SEQ ID NO: 5)
(in SEQ ID NO: 5, N₂₆'s each independently represent any base, m is an integer of 1 or more, (N₂₆)ₘ forms a T-loop, and GGCGG forms a T-stem with CCGCU)
GGAGG(N₂₇)ₘCCUCU (SEQ ID NO: 6)
(in SEQ ID NO: 6, N₂₇'s each independently represent any base, m is an integer of 1 or more, (N₂₇)ₘ forms a T-loop, and GGAGG forms a T-stem with CCUCU).

6. The production method according to Claim 1,
wherein the initiator tRNA further comprises a nucleotide sequence set forth in SEQ ID NO: 7,
N₂₈GN₂₉UC (SEQ ID NO: 7)
(in SEQ ID NO: 7, N₂₈ represents A or G, N₂₉ represents A or G, and N₂₈GN₂₉UC forms a variable loop).

7. The initiator tRNA according to any one of Claims 1 to 6.

8. A translation system comprising:
the initiator tRNA according to Claim 7.

9. A method for producing a peptide library, comprising:
a step of translating in a cell-free translation system using the translation system according to Claim 8.

10. A method for producing a peptide-mRNA complex library, which is a library of a complex of a peptide with an mRNA encoding the peptide, the method comprising:
a step of translating in a cell-free translation system using the translation system according to Claim 8.

11. A peptide library produced by the method according to Claim 9.

12. A peptide-mRNA complex library produced by the method according to Claim 10.

13. The peptide library according to Claim 11,
wherein the peptide library comprises a peptide comprising, at an N terminus, a D-amino acid, a β-amino acid, a γ-amino acid, or L-proline, each comprising a nitrogen atom modified at an α-position, a β-position, or a γ-position.

14. The peptide-mRNA complex library according to Claim 12,
wherein the peptide-mRNA complex library comprises a complex of a peptide comprising, at an N terminus, a D-amino acid, a β-amino acid, a γ-amino acid, or L-proline, each comprising a nitrogen atom modified at an α-position, a β-position, or a γ-position, and an mRNA encoding the peptide.
